(19) Europäisches Patentamt
European Patent Office
Office européen des brevets

(11) **EP 1 719 410 B1**

(12) **EUROPEAN PATENT SPECIFICATION**

(45) Date of publication and mention of the grant of the patent:
**14.07.2010 Bulletin 2010/28**

(21) Application number: **05710694.0**

(22) Date of filing: **18.02.2005**

(51) Int Cl.:
***A01N 63/02*** *(2006.01)*

(86) International application number:
**PCT/JP2005/003094**

(87) International publication number:
**WO 2005/082149 (09.09.2005 Gazette 2005/36)**

(54) **Method of controlling plant disease damage by using a bacillus**

Verfahren zur Bekämpfung von Pflanzenkrankheitsschäden durch Verwendung eines Bacillus

Procédé de lutte contre les dommages causés par les maladies des plantes utilisant un bacille

(84) Designated Contracting States:
**AT BE BG CH CY CZ DE DK EE ES FI FR GB GR HU IE IS IT LI LT LU MC NL PL PT RO SE SI SK TR**

(30) Priority: **27.02.2004 JP 2004055059**
**23.07.2004 JP 2004216083**

(43) Date of publication of application:
**08.11.2006 Bulletin 2006/45**

(73) Proprietor: **Itsuki Co., Ltd.**
**Asaka-shi, Saitama 3510015 (JP)**

(72) Inventors:
- **YUKI, Daiju,**
**Itsuki Co., Ltd.**
**Asaka-shi,**
**Saitama 3510015 (JP)**
- **KISO, Akira,**
**Itsuki Co., Ltd.**
**Asaka-shi,**
**Saitama 3510015 (JP)**

(74) Representative: **Denison, Christopher Marcus et al**
**Mewburn Ellis LLP**
**33 Gutter Lane**
**London**
**EC2V 8AS (GB)**

(56) References cited:

| | |
|---|---|
| **WO-A1-00/49875** | **WO-A1-03/046157** |
| **JP-A- 2 209 803** | **JP-A- 5 051 305** |
| **JP-A- 5 091 869** | **JP-A- 6 133 763** |
| **JP-A- 10 146 187** | **JP-A- 2001 346 407** |
| **JP-A- 2003 277 210** | **JP-A- 2003 289 854** |
| **JP-A- 2005 000 145** | **US-A1- 2003 082 792** |

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 719 410 B1

## Description

Technical Field

The present invention relates to a method for controlling bacterial, fungal, or viral plant diseases and a control agent or material therefor.

Background Art

In the agricultural cultivation of cash crops such as vegetables, development of seed-borne, soil-borne, or air- or water-borne plant diseases often involves complications in terms of the production of high-quality crops, stable production, and profitable earnings. Particularly when faced with abnormal weather conditions where plant diseases are likely to occur, commercial farms will be fatally affected.

The main pathogens that are recognized as causal factors of such transmissible diseases are filamentous fungi, bacteria, and viruses. In order to realize normal cultivation, it is indispensable that plant diseases caused by such pathogens be controlled.

In order to effectively protect agricultural products from diseases, chemical techniques are extensively employed, such as application of pesticides (e.g., fungicides, bactericides, or antiviral agents). In recent years, synthetic organic pesticides have become remarkably widespread because of their effects, and such agents have effectively controlled plant diseases, which has resulted in remarkably improved production of agricultural products. However, disease control that relies heavily on the use of pesticides involves global environmental, social, and agricultural disadvantages, such as residual pesticides in soils, residual pesticides in foodstuffs, and increased drug resistance of pathogenic microorganisms. Also, excess use of pesticides is problematic in respect of the safety of farmers.

These issues have received a great deal of attention worldwide, and a variety of measures are being attempted in response. For example, the Montreal Protocol banned the use of a soil fumigant, i.e., methyl bromide, in 2005, except for crucial use, as it is a cause of ozone layer depletion.

As an alternative to excessive dependence on pesticides, establishment of a system of integrated pest management (IPM), which is in harmony with the natural ecosystem, is strongly desired worldwide.

A method of controlling plant diseases with the utilization of the abilities of microorganisms such as bacteria, i.e., a means of disease control involving the use of microbial pesticides, is expected as an effective means to resolve the aforementioned problem.

The following prior art documents are available concerning the invention of the present application, for example. US Patent No. 5,344,647; US Patent No. 5,061,495; JP Patent Publication (Unexamined) No. 8-175919; JP Patent Publication (Unexamined) No. 2003-277210; JP Patent Publication (Unexamined) No. 2002-145712; JP Patent Publication (Unexamined) No. 9-124427; JP Patent Publication (Unexamined) No. 11-302120; US Patent No. 5,935,571; JP Patent Publication (Unexamined) No. 7-267811; Phae, C. G., Shoda, M., Kita, N., Nakano, M., and Ushiyama, K., Ann. Phytopath. Soc. Japan, 58, 329-339, 1992; Masao Yamada, Biseibutsu nouyaku - kankyou hozenkata nougyou wo mezasite (Microbial pesticides -in pursuit of environmental conservative agriculture), Zenkoku nouson kyouiku kyoukai, p. 328, 2000; and Shunichi Iwata et al. (ed.), Shokubutsu boueki kouza-byougai-hen (Plant protection course - disease volume, Japan Plant Protection Association, p. 281, 1983.

Disclosure of the Invention

The present invention relates to a control agent or material for biologically controlling plant diseases caused by plant pathogenic bacteria, fungi, or viruses, a method for controlling plant diseases using the same, and bacteria that can be used therefor, all as defined in the claims.

The DAIJU-SIID2550 strain was deposited at the International Patent Organism Depositary of the National Institute of Advanced Industrial Science and Technology (Tsukuba Central 6, 1-1-1 Higashi, Tsukuba, Ibaraki, 305-8566, Japan) as of November 20, 2003, under the accession number: FERM BP-10114 (transferred from the FERM P-19591 strain that had been domestically deposited as of November 20, 2003). This deposit was made by an inventor of the present invention, Daiju Yuki (3-10-5, Sakae Niiza, Saitama, 352-0014, Japan), on November 20, 2003, and transferred to the applicant of the present application, Itsuki Co., Ltd. (3-12-5, Saiwai-cho, Asaka, Saitama, 351-0015, Japan), prior to filing, i.e., February 7, 2005.

According to the present invention, plant diseases caused by infection with or proliferation of plant pathogenic bacteria, fungi, or viruses can be biologically controlled.

Brief Description of Drawings

Fig. 1 shows the effects of inhibiting proliferation of *Xanthomonas campestris pv. campestris* via BAL bacteria.
Fig. 2 shows the conditions of *Xanthomonas campestris pv. campestris* development upon germination of bok choy seeds artificially contaminated with *Xanthomonas campestris pv. campestris.*
Fig. 3 shows the conditions of *Xanthomonas campestris pv. campestris* growth in the vicinity of seeds artificially contaminated with *Xanthomonas campestris pv. campestris,* which had been treated with a BAL bacteria-containing control agent.
Fig. 4 shows the appearance of samples 9 days after the initiation of the experiment of Example 8.
Fig. 5 shows the appearance of samples 9 days after the initiation of the experiment of Example 8.
Fig. 6 shows the appearance of samples 26 days after the initiation of the experiment of Example 9.
Fig. 7A shows the influence of a bactericide and an insecticide on proliferation of BAL bacteria.
Fig. 7B shows the influence of a bactericide and an insecticide on proliferation of BAL bacteria (continuation of Fig. 7A).
Fig. 8 shows the effects of inhibiting the proliferation of canker bacteria via BAL bacteria.
Fig. 9 shows the effects of inhibiting the proliferation of wilt bacteria via BAL bacteria.
Fig. 10 shows the effects of inhibiting the proliferation of soft rot bacteria via BAL bacteria.
Fig. 11 shows the appearance of samples 1 day after the initiation of an experiment of Example 14.
Fig. 12A shows the appearance of samples 16 days after the initiation of an experiment concerning damping-off of rice seedlings of Example 15.
Fig. 12B shows the appearance of samples 16 days after the initiation of an experiment concerning bacterial grain rot of rice of Example 15. -
Fig. 13 shows the appearance of samples 24 hours after the initiation of an experiment of Example 16. The multiplying factors in parenthesis indicate the final dilution factors at the time of germination testing.
Fig. 14 shows the appearance of samples 24 hours after the initiation of an experiment of Example 17.
Fig. 15 shows the effects of a culture solution of BAL bacteria on gray mold of Example 19.
Fig. 16 shows the appearance of samples 20 days after the initiation of an experiment of Example 20.
Fig. 17 shows the effects of a culture solution of BAL bacteria on black spot of Example 21.
Fig. 18 shows the effects of a culture solution of BAL bacteria on white rust of Example 22.
Fig. 19 shows the appearance of samples 18 days after the initiation of an experiment of Example 23.
Fig. 20 shows the appearance of samples 11 days after the initiation of an experiment of Example 24.
Fig. 21 shows the effects of a culture solution of BAL bacteria on TMV of Example 25.

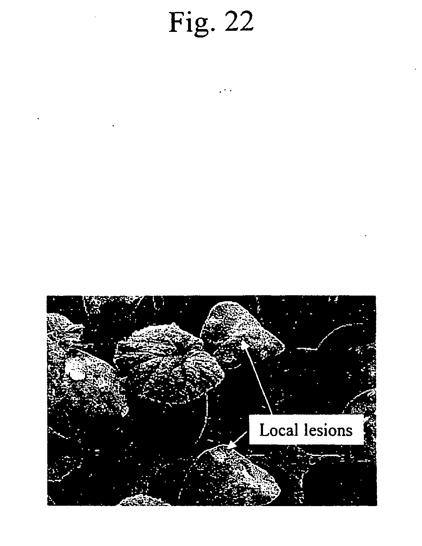

Fig. 22 shows the effects of a culture solution of BAL bacteria on melon necrotic spot of Example 26.

Preferred Embodiments of the Invention

The present invention relates to a method for controlling plant diseases comprising application of bacteria of the genus *Bacillus* capable of inhibiting infection with or proliferation of plant pathogenic bacteria, fungi, or viruses to plants that serve as hosts of plant pathogenic bacteria, fungi, or viruses, wherein the bacteria of the genus Bacillus are of the DAIJU-SIID2550 strain (accession number FERM BP-10114).
The present invention also relates to a control agent or material for plant diseases comprising bacteria of the genus *Bacillus* capable of inhibiting infection with or proliferation of plant pathogenic bacteria, fungi, or viruses, wherein the bacteria of the genus Bacillus are of the DAIJU-SIID2550 strain (accession number FERM BP-10114).
Further, the present invention relates to the use of bacteria of the genus *Bacillus* capable of inhibiting infection with or proliferation of plant pathogenic bacteria, fungi, or viruses for the production of a control agent or material for plant diseases caused by bacteria, fungi, or viruses, wherein the bacteria of the genus Bacillus are of the DAIJU-SIID2550 strain (accession number FERM BP-10114).
The present invention provides a method for biologically controlling plant diseases caused by plant pathogenic bacteria, fungi, or viruses and a control agent and a control material therefor. Up to the present, several methods for controlling plant diseases with bacteria of the genus *Bacillus* have been developed (see the documents described in the "Background Art" section above); however, none of these methods is equivalent to the invention disclosed by the present invention.
Bacterial plant diseases controlled by the present invention are any plant diseases caused by bacterial pathogens. Examples thereof include plant diseases caused by bacteria of the genera *Agrobacterium, Clavibacter, Erwinia, Pseudomonas, Ralstonia, Corynebacterium, Curtobacterium, Burkholderia, Xanthomonas, Rhizobacter,* and *Clostridium.* The control agent or material of the present invention is particularly useful on plant diseases caused by bacteria of the genus *Agrobacterium, Clavibacter, Erwinia, Pseudomonas, Ralstonia, Corynebacterium, Curtobacterium, Burkhol-*

*deria,* or *Xanthomonas.* Specific examples include, but are not limited to, black rot caused by infection of *Brassicaceae* plants by *Xanthomonas campestris pv. campestris* (it refers to black rot bacteria in this description), hairy root caused by infection of melon by *Agrobacterium rhizogenes,* crown gall caused by infection of carrot by *Agrobacterium tumefaciens,* scale rot caused by infection of onion by *Burkholderia gladioli,* bacterial canker caused by infection of tomato by *Clavibacter michiganensis subsp. michiganensis,* bacterial canker caused by infection of capsicum and bell pepper by bacteria of *Corynebacterium sp.,* bacterial leaf gall caused by infection of tomato by bacteria of *Corynebacterium sp.,* bacterial canker caused by infection of onion by *Curtobacterium flaccumfaciens,* soft rots (e.g., bacterial soft rot and slimy soft rot) caused by infection of *Brassica campestris, Brassica campestris L. var. amplexicaulis Tanaka et Ono, Brassica campestris L. var. chinensis (L.) Ito,* turnip, cauliflower, cabbage, cucumber, Japanese radish, pak choi, onion, capsicum, bell pepper, tomato, eggplant, *Allium tuberosum,* carrot, *Allium sativum, Allium fistulosum,* Chinese cabbage, broccoli, melon, or lettuce by *Erwinia carotovora subsp. carotovora,* bacterial stem rot caused by infection of strawberry by *Erwinia chrysanthemi,* bacterial rot caused by infection of pumpkin by *Erwinia chrysanthemi,* bacterial stem rot caused by infection of eggplant by *Erwinia chrysanthemi,* bacterial soft rot caused by infection of *Allium fistulosum* by *Erwinia chrysanthemi,* bacterial rot (soft rot) caused by infection of onion by *Erwinia rhapontici,* rot caused by infection of *Allium sativum* by bacteria of *Erwinia sp.,* bacterial leaf blight caused by infection of okra by *Pseudomonas cichorii,* necrotic leaf spot caused by infection of eggplant by *Pseudomonas cichorii,* rot caused by infection of *Allium sativum* by *Pseudomonas cichorii,* bacterial rot caused by infection of melon and lettuce by *Pseudomonas cichorii,* pith necrosis caused by infection of tomato and eggplant by *Pseudomonas corrugata,* pith necrosis caused by infection of tomato by *Pseudomonas fluorescens,* bacterial bud rot caused by infection of *Brassica campestris var. pekinensis* by *Pseudomonas marginalis,* bud blight caused by infection of strawberry by *Pseudomonas marginalis pv. marginalis,* bacterial rots (e.g., soft rot and head rot) caused by infection of cabbage, *Chrysanthemum coronarium,* onion, *Allium fistulosum,* Chinese cabbage, broccoli, and lettuce by *Pseudomonas marginalis pv. marginalis,* marginal blight caused by infection of cucumber by *Pseudomonas marginalis pv. marginalis,* black ring rot caused by infection of Japanese radish by *Pseudomonas marginalis pv. marginalis,* bacterial rot caused by infection of tomato by *Pseudomonas marginalis pv. marginalis,* rot caused by infection of *Allium sativum* by *Pseudomonas marginalis pv. marginalis,* brown rot caused by infection of strawberry by bacteria of *Pseudomonas sp.,* Tomato black spot caused by infection of tomato by bacteria of *Pseudomonas sp.,* bacterial spot caused by infection of eggplant by bacteria of *Pseudomonas sp.,* bacterial basal bulb rot caused by infection of *Allium tuberosum* by bacteria of *Pseudomonas sp.,* bacterial spot (bacterial leaf spot) caused by infection of onion and *Allium fistulosum* by *Pseudomonas syringae,* bacterial spots (e.g., angular leaf spot and bacterial spot) caused by infection of pumpkin, cucumber, *Momordica charantia,* and melon by *Pseudomonas syringae pv. lachrymans,* bacterial black spots (e.g., bacterial leaf spot and bacterial black spot) caused by infection of *Brassica campestris, Brassica campestris L. var. amplexicaulis Tanaka et Ono, Brassica campestris L. var. chinensis (L.) Ito,* turnip, *Brassica juncea Brassica juncea var. integlifolia,* cauliflower, cabbage, *Brassica rapa var. laciniifolia,* Japanese radish, and Chinese cabbage by *Pseudomonas syringae pv. maculicola,* bacterial spot (bacterial leaf spot) caused by infection of spinach by *Pseudomonas syringae pv. spinaciae,* bacterial speck caused by infection of tomato by *Pseudomonas syringae pv. tomato,* bacterial leaf blight caused by infection of okra by *Pseudomonas viridiflava,* bacterial rot (soft rot) caused by infection of cabbage by *Pseudomonas viridiflava,* marginal blight caused by infection of cucumber by *Pseudomonas viridiflava,* bacterial black spot caused by infection of tomato by *Pseudomonas viridiflava,* bacterial bud rot caused by infection of *Brassica campestris var. pekinensis* by *Pseudomonas viridiflava,* bacterial brown streak caused by infection of Chinese cabbage by *Pseudomonas viridiflava,* bacterial rot caused by infection of lettuce by *Pseudomonas viridiflava,* bacterial wilt caused by infection of strawberry, turnip, pumpkin, cucumber, Chrysanthemum coronarium, Japanese radish, capsicum, bell pepper, tomato, eggplant, and *Momordica charantia* by *Ralstonia solanacearum,* bacterial gall caused by infection of carrot by *Rhizobacter dauci,* bacterial angular leaf spot caused by infection of strawberry by *Xanthomonas campestris,* black rot caused by infection of *Chrysanthemum coronarium* by *Xanthomonas campestris,* black rot caused by infection of broccoli by *Xanthomonas campestris,* bacterial leaf blight caused by infection of *Allium fistulosum* by *Xanthomonas campestris pv. allii,* bacterial spot (bacterial blight) caused by infection of carrot by *Xanthomonas campestris pv. carotae,* necrotic leaf spots (e.g., bacterial spot, bacterial brown spot, and bacterial leaf spot) caused by infection of pumpkin, cucumber, and melon by *Xanihomonas campestris pv. cucurbitae,* bacterial spot caused by infection of turnip, cabbage, Japanese radish, bok choy, and Chinese cabbage by *Xanthomonas campestris pv. raphani,* bacterial spot caused by infection of capsicum, bell pepper, and tomato by *Xanthomonas campestris pv. vesicatoria,* bacterial spot caused by infection of lettuce by *Xanthomonas campestris pv. vitians,* bacterial angular leaf spot caused by infection of strawberry by *Xanthomonas fragariae,* halo blight caused by infection of rice by *Pseudomonas syringae pv. oryzae,* bacterial brown stripe caused by infection of rice by *Acidovorax avenae subsp. avenae,* bacterial foot rot caused by infection of rice by *Erwinia chrysanthemi pv. zeae,* bacterial leaf blight caused by infection of rice by *Xanthomonas oryzae pv. oryzae,* bacterial palea browning caused by infection of rice by *Erwinia ananas,* bacterial seedling blight caused by infection of rice by *Burkholderia plantarii,* bacterial grain rot caused by infection of rice by *Burkholderia glumae,* sheath brown rot caused by infection of rice by *Pseudomonas fuscovaginae,* bacterial black node caused by infection of wheat by *Pseudomonas syringae pv. japonica,* bacterial wilt caused by infection of potato by *Ralstonia solanacearum,* bacterial stem rot caused by infection of potato by *Erwinia chrysanthemi pv. chrysanthemi,* black leg caused by infection of potato by *Erwinia carotovora subsp. atroseptica, Erwinia carotovora subsp. carotovora,* and *Erwinia chrysanthemi,* Bacterial soft rot caused by infection of potato by *Erwinia carotovora subsp. carotovora,* slimy rot caused by infection of potato by bacteria of *Clostridium sp.,* ring rot caused by infection of potato by *Clavibacter michiganensis subsp. sepedonicus,* bacterial pustule caused by infection of soybean by *Xanthomonas campestris pv. glycines,* bacterial spot (bacterial blight) caused by infection of soybean by *Pseudomonas syringae pv. glycinea,* bacterial brown stripe caused by infection of corn by *Acidovorax avenae subsp. avenae,* bacterial stripe caused by infection of corn by *Burkholderia andropogonis,* and bacterial stalk rot caused by infection of corn by *Erwinia chrysanthemi pv. zeae* and *Pseudomonas marginalis.*

Black rot caused by *Xanthomonas campestris pv. campestris* is known as a disease that fatally interferes the production of *Brassicaceae* plants. As a critical disease, the control of black rot has received a great deal of attention from all over the world, including the nations where black rot has already occurred and the nations where black rot has not occurred yet, as well as in Japan. Up to the present, there is no plant variety that has been practically approved to be resistant to black rot, and any effective pesticide has not yet been developed. According to the present invention, black rot of *Brassicaceae* plants, such as cabbage, Chinese cabbage, Japanese radish, broccoli, cauliflower, turnip, bok choy, *Brassica chinensis komatsuna, Brassica campestris L. var. chinensis (L.) Ito, Brassica napus,* Shirakukina, *Brassica campestris L. var. amplexicaulis Tanaka et Ono, Brassica campestris, Brussels sprouts, kohlrabi,* Chinese kale, *Brassica chinensis var. rosularis, Brassica juncea, Brassica campestris,* and cabbage *var. acephala* can be effectively controlled.

Bacterial grain rot of rice and bacterial seedling blight of rice are seed-borne diseases from rice chaff, they must be controlled in wetland rice cultivation, they are critical bacterial diseases of rice, and seed disinfection is particularly indispensable. To this end, a technique of chemical control is currently put to practical use in general. Disinfection of rice chaff with pesticides, however, involves the problem of a treatment of residual pesticides and the like. "Momigenki" (Nissan Chemical Industries, Ltd.) is the only commercially available biotic pesticide for disinfecting rice seed. According to the present invention, seed-borne bacterial diseases of wetland rice can be effectively controlled.

Fungal diseases that are controlled by the present invention may be any plant diseases caused by fungal pathogens. Examples thereof include airborne fungal diseases and soil-borne fungal diseases. In the present invention, airborne fungal diseases include waterborne diseases. Examples of airborne fungi include, but are not limited to, powdery mildews (the genera *Erysiphe, Sphaerotheca,* and *Leveillula*), the genus *Botrytis,* the genus *Fulvia fulva,* the genus *Corynespora,* the genus *Albugo,* downy mildews (the genera of *Pseudoperonospora, Peronospora, Plasmopara,* and *Bremia),* blast fungi (the genus *Pyricularia grisea), Cochliobolus miyabeanus, Cercospora* and related genera (the genera *Cercospora, Cercosporella, Pseudocercospora, Paracercospora,* and *Mycovellosiella*), *Bacillus anthrasis* (the genus *Colletotrichum* and *Glomerella*), rust fungi (the genus *Puccinia*), black spot fungus (the genus *Alternaria*), and the genus *Alternaria.* Examples of soil-borne fungi include, but are not limited to, the club root fungus (*Plasmodiophora brassicae*), the genus *Phytophthora,* the genus *Fusarium,* the genus *Verticillium,* the genus *Pythium,* and the genus *Rhizoctonia.*

Specific examples of airborne fungal diseases include, but are not limited to: powdery mildew caused by infection of tomato, eggplant, Japanese radish, cabbage, turnip, *Brassica juncea,* Chinese cabbage, *Brassica chinensis komatsuna,* carrot, cucumber, strawberry, capsicum, melon, watermelon, pumpkin, and chrysanthemum by powdery mildews; gray mold caused by infection of *Cucurbitaceae, Solanaceae,* lettuce, pulses, and strawberry by *Botrytis cinerea; Corynespora* leaf spot caused by infection of cucumber by *Corynespora cassiicola;* white rust caused by infection of a variety of *Brassicaceae* vegetables, such as Chinese cabbage, cauliflower, turnip, Japanese radish, and *Brassica chinensis komatsuna* by *Albugo macrospora;* downy mildew caused by infection of gourd, *Brassicaceae* vegetables, *Allium fistulosum,* onion, horseradish, *Chrysanthemum coronarium*, *Cryptotaenia japonica,* spinach, and lettuce by downey mildews; rice blast caused by infection of rice by blast fungus; tomato leaf mold (Cercospora leaf mold) caused by infection of tomato by *Pseudocercospora fuligena;* eggplant brown round spot (leaf spot) caused by infection of eggplant by *Paracercospora egenula;* spot plant disease (frogeye leaf spot) caused by infection of bell pepper and capsicum by *Cercospora capsici;* spot plant disease (leaf spot) caused by infection of cucumber, melon, watermelon, and loofah by *Cercospora citrullina;* white spot on Chinese cabbage and turnip caused by infection of Chinese cabbage and turnip by *Cercosporella brassicae;* eggplant leaf mold caused by infection of eggplant by *Mycovellosiella nattrassii;* anthracnose caused by infection of Chinese cabbage, turnip, Japanese radish, *Brassica chinensis komatsuna, Beta vulgaris,* bok choy, *Brassica campestris,* bush bean, capsicum, bell pepper, gourd, green soybean, *Allium fistulosum,* onion, *Allium tuberosum,* spinach, and strawberry by *Bacillus anthrasis;* rust caused by infection of *Allium fistulosum,* onion, *Allium bakeri, Allium sativum,* and *Allium tuberosum by* rust fungi; chrysanthemum rust caused by infection of chrysanthemum by rust fungi; black spot (e.g., *Alternaria* leaf spot and *Alternaria* black rot) caused by infection of *Brassicaceae* vegetables and carrot by black spot fungi; *Alternaria* sooty spot caused by infection of cabbage by *Alternaria brassicicola;* leaf blight caused by infection of carrot by *Alternaria dauci;* early blight caused by infection of potato by *Alternaria solani;* tomato early blight caused by infection of tomato by *Alternaria solani;* leaf blight caused by infection of onion and *Allium tuberosum* by a fungus of the genus Botrytis; and gray-mold neck rot caused by infection of onion by *Botrytis allii.* Airborne fungal diseases, for example, white rust, black spot, anthracnose, and downy mildew, are known to fatally interfere with the production of leaf and root vegetables of *Brassicaceae,* and the control of such serious diseases has received a great deal of attention from all over the world, including the nations where such diseases have already occurred and the nations where such diseases have not occurred yet, as well as in Japan. Up to the present, there is no plant variety that has been practically approved to be resistant to white rust, black spot, anthracnose, and downy mildew. There are some pesticides with sufficient controlling effects (e.g., metalaxyl wettable powder, iprodione wettable powder, TPN wettable powder, and thiophanate-methyl wettable powder). Since these pesticides are directly applied to leaf and root vegetables for human consumption, the standard for safe use is strict, and use of such pesticides may produce adverse impacts during the disease control. The present invention enables biological control of such diseases of, for example, turnip, bok choy, *Brassica chinensis komatsuna, Brassica campestris L. var. chinensis (L.) Ito,* Japanese radish, Chinese kale, *Brassica chinensis var. rosularis,* cauliflower, broccoli, Chinese cabbage, cabbage, *Brassica campestris,* and *Brassica juncea* (see Examples 21 and 22).

Specific examples of soil-borne fungal diseases include, but are not limited to: clubroot caused by infection of *Brassicaceae* crops by *Plasmodiophora brassicae; Phytophthora*-induced diseases, such as blight caused by infection of rice, apple, lily, strawberry, citrus, pear, bell pepper, capsicum, pumpkin, colocasia, potato, tomato, eggplant, cucumber, watermelon, melon, gourd, *Allium fistulosum, Dianthus caryophyllus,* citrus, and spinach, gray blight caused by infection of tomato and cucumber, brown rot caused by infection of eggplant, tomato, watermelon, and citrus, root rot caused by infection of strawberry, a yellow dwarf disease caused by infection of rice, barley, wheat, *Avena sativa,* corn, and bentgrass; stem rot caused by infection of roses, cotton blight caused by infection of rice, soybean, and eggplant, foot rot caused by infection of citrus, white blight caused by infection of onion, *Allium fistulosum, Allium fistulosum caespitosum, Allium bakeri, Allium sativum, Allium tuberosum,* and *Allium grayi,* stem blight caused by infection of *Phaseolus angularis* and soybean, seedling and leaf blight caused by infection of eggplant, and root rot caused by infection of tomato and eggplant; Pythium-induced diseases, such as cottony blight caused by infection of bush bean, pumpkin, and cucumber, seedling damping-off caused by infection of rice, eggplant, tomato, spinach, pumpkin, cucumber, bush bean, garden pea, okra, and melon, cavity spot caused by infection of carrot, Pythium blight caused by infection of bentgrass, damping-off caused by infection of soybean, cucumber, melon, and spinach, brown snow mold caused by infection of wheat, barley, *Avena sativa,* rye, ryegrass, and fescue, root rot caused by infection of strawberry, cucumber, colocasia, and tomato, white rot caused by infection of sweet potato, and seedling rot caused by infection of rice; Rhizoctonia-induced diseases, such as red crown rot caused by infection of rice, sheath blight caused by infection of rice, barley, *Setaria italica,* Sorghum bicolor, and millet, leaf rot caused by infection of soybean, *Phaseolus angularis,* bush bean, garden pea, potato, orchardgrass, bluegrass, fescue, ryegrass, bentgrass, and sorghum, bud blight caused by infection of strawberry, stem rot caused by infection of broad beans and *Dianthus caryophyllus,* seedling damping-off caused by infection of rice, tomato, eggplant, bell pepper, capsicum, cucumber, melon, *Cucumis melo, Lagenaria siceraria*, cabbage, cauliflower, broccoli, *Brassica chinensis komatsuna,* onion, *Allium fistulosum*, okra, and *Cyclamen*, seedling blight caused by infection of spinach, barley, wheat, and cabbage, foot blight caused by infection of lettuce, black scurf caused by infection of potato and burdock, root rot caused by infection of Japanese radish and carrot, bottom rot caused by infection of Chinese cabbage, violet root rot caused by infection of carrot, and damping-off caused by infection of chrysanthemum; *Verticillium*-induced diseases, such as wilt caused by infection of strawberry, *Aralia cordata,* and soybean, *Verticillium* wilt caused by infection of eggplant, capsicum (bell pepper), tomato, okra, cucumber, *Petasites japonica,* melon, chrysanthemum, and rose, *Verticillium* wilt caused by infection of cabbage and lettuce, *Verticillium* black-spot disease caused by infection of Japanese radish, and icterus caused by infection of Chinese cabbage; and *Fusarium*-induced diseases, such as Fusarium wilt caused by infection of eggplant, Fusarium wilt caused by infection of cucumber, *Lagenaria siceraria,* loofah, melon, and watermelon, Stem rot caused by infection of sweet potato, yellows caused by infection of cabbage, strawberry, Japanese radish, turnip, *Brassica chinensis komatsuna,* and *Aralia cordata,* root rot caused by infection of lettuce and bush bean, dry rot caused by infection of *Allium bakeri,* onion, *Allium sativum, Allium tuberosum,* colocasia, and carrot, seedling blight caused by infection of *Cryptotaenia japonica,* wilt caused by infection of burdock, *Allium fistulosum,* tomato, spinach, *Dianthus caryophyllus, Cyclamen,* and *Phaseolus angularis,* damping-off caused by infection of asparagus and *Dianthus caryophyllus,* bacterial rot caused by infection of lotus, brown rot caused by infection of melon and yam, *Fusarium* crown and root rot caused by infection of tomato and *Allium fistulosum,* seedling rot caused by infection of rice, and pink snow mold caused by infection of wheat. Soil-borne fungal diseases caused by, for example, *Fusarium, Verticillium,* or *Phytophthora* fungi are difficult to control and these diseases fatally affect the cultivation of vegetables. In order to deal with such diseases, cultivation of resistant varieties, disinfection of soil with chemical pesticides, disease forecasting, and other attempts have been made. For example, cultivation of resistant varieties or rootstocks of *Solanaceae* (e.g., tomato, eggplant, bell pepper, and green pepper) and *Cucurbitaceae* (e.g., cucumber, watermelon, and melon), disinfection of soils with chemical pesticides (e.g., chloropicrin, dazomet, and methyl bromide), or soil testing for pathogens have been performed in order to control the diseases. The harvest period of *Brassicaceae* vegetables, such as turnip, bok choy, *Brassica chinensis komatsuna,* Chinese cabbage, cabbage, or Japanese radish, is shorter than that of vegetables of *Solanaceae* or *Cucurbitaceae.* Accordingly, the frequency of continuous cropping is very high per year. For example, the number of continuous cropping of *Brassica chinensis komatsuna,* bok choy, or turnip is 5 or 6 per year. Thus, the damage resulting from soil-borne diseases is fatal. Use of the control agent or material of the present invention in every cropping season enables prevention of proliferation or transmission of pathogens that are hard to control in soil and reduction of development of diseases (see Examples 23 and 24).

Viral plant diseases that are controlled by the present invention can be any plant diseases caused by viral pathogens. Examples thereof include, but are not limited to, plant diseases caused by *Tobacco mosaic virus* (TMV), *Pepper mild mottle virus* (PMMoV), *Tomato mosaic virus* (ToMV), *Melon necrotic spot virus* (MNSV), *Cucumber green mottle mosaic virus* (CGMMV), or *Kyuri green mottle mosaic virus* (KGMMV).

Specific examples of viral plant diseases include, but are not limited to, tobacco mosaic disease caused by TMV, bell pepper mosaic disease caused by PMMoV, ToMV, or TMV, melon necrotic spot caused by MNSV, cucumber green mottle mosaic disease caused by CGMMV, melon green mottle mosaic disease caused by CGMMV, and kyuri green mottle mosaic virus disease caused by KGMMV.

Viral diseases caused by TMV, KGMMV, CGMMV, and MNSV exhibit more potent contact infectivity, sap transmissibility, seed transmissibility, and soil transmissibility than diseases caused by other viruses. Thus, the control of such critical viral disease has received a great deal of attention from all over the world, plant varieties or rootstocks resistant to such diseases have been cultivated, and such cultivation products have been put to practical use. In some cases, the market or consumer demand surpasses the cultivation capacity, and effective resistant varieties or rootstocks may not be used. Thus, a technique of chemical control is employed particularly for the prevention of sap transmission, seed transmission, and soil transmission. For example, prevention of sap transmission with the use of Lentemin water-soluble powders, prevention of seed transmission and sap transmission with the use of trisodium phosphate, or prevention of soil transmission with the use of methyl bromide has been performed (see Iwata et al., above). Since these control techniques are implemented via chemical treatments, misuse may result in unsatisfactory effects, crop injury, or human suffering. Further, methyl bromide (a methyl bromide fumigant) had been extensively used in Japan in order to control soil transmission of viral diseases. Use of this fumigant was banned in 2005 under the Montreal Protocol, because of its correlation with ozone layer depletion. At present, alternatives thereto are being extensively searched for. According to the present invention, seed-borne viral diseases and soil-borne viral diseases can be effectively controlled by biological means (see Examples 25 and 26).

Examples thereof include DAIJU-SIID2550 (accession number: FERM BP-10114) is deduced to be related to *Bacillus amyloliquefaciens,* and thus is hereafter abbreviated as "BAL bacteria." An agent or material for plant disease control containing BAL bacteria is used, since it is capable of firmly colonizing a plant or soil, efficiently sustaining the activity of plant disease control, and maintaining the activity of disease control even if the agent or material is exposed to a high temperature between 40°C and 100°C.

The aforementioned BAL bacteria can be isolated in the following manner, for example. The residue of edible mushroom culture is subjected to steam sterilization at 80°C for 30 minutes, the residue is diluted with the addition of physiological saline at a ratio of 1:9 (residue: physiological saline) by weight, and the diluent is shaken using a reciprocating shaker with an amplitude of 10 cm for 15 minutes. Thereafter, the product is subjected to low-speed centrifugation at 500 rpm for 5 minutes to obtain a supernatant. Alternatively, the product is subjected to filtration with Toyo Roshi No. 2 to obtain a filtrate. Subsequently, the supernatant or filtrate is serially diluted with distilled water containing 0.05% agar (*"Saikingaku jisshu teian* (Proposal regarding bacteriological laboratory training)," the Alumni Association of the Institute of Infectious Diseases (ed.)). Serial dilution is carried out by preparing $1 \times 10^{1-8}$-fold diluents, dispensing 100 μl each of the diluents into YPA medium (a medium containing peptone, yeast, and salt; see *"Shokubutsu byougensei biseibutsu kenkyu-hou (Study of plant pathogenic microorganisms), idenshi sousa wo fukumu kiso to ouyo (Basics and applications including genetic engineering),"* Satoshi Wakimoto (ed.)) in a 9-cm petri dish, performing static culture in an incubator at 25°C for 48 to 72 hours, and separately sampling the grown bacteria. The sampled bacteria are then subjected to passage culture in YPA slant medium. In order to confirm that the bacteria are not contaminated with irrelevant bacteria, the passage-cultured bacteria are subjected to serial dilution again to obtain single colonies. From among the pure single colonies of bacteria thus separated, the bacteria having the most potent capacity for inhibition are selected based on the intensity of the inhibition zone formed via confrontation culture on a YPA plate of bacteria of each single colony and turnip yellows viruses (*Fusarium oxysporum f. sp. conglutinans,* a simple marker for confirming the antimicrobial activity against *Xanthomonas campestris pv. campestris*).

The thus-selected bacteria have the following properties: gram stainability: positive; endospore: present; morphology: rod-shaped; G+C (DNA) content (mol%): 43.5 to 44.9 (this value is higher than that for *B. subtilis,* which is 42 to 43); optimal temperature for growth: 25°C to 30°C; and biosafety level: 1 (which would not cause disease in humans and would not cause serious disease in plants or animals).

The taxonomic group of the bacteria was deduced with the use of the approximately 1600-bp-long 16S rDNA nucleotide sequence (the 16S rRNA gene) (see the following for details). As a result, the bacteria of interest were found to be of a novel strain of the genus *Bacillus.* The bacteria of interest are deposited at the International Patent Organism Depositary of the National Institute of Advanced Industrial Science and Technology as of November 20, 2003, as the DAIJU-SIID2550 strain (accession number: FERM BP-10114)

BAL bacteria can be cultured by conventional culture techniques of liquid culture, such as reciprocal shake culture, jar fermentor culture, or culture with the use of a culture tank, or solid culture. Culture media for BAL bacteria are not particularly limited, as long as the bacteria can effectively reach the logarithmic growth phase. With the use of a preferable medium, the maximal yield can be attained at 25°C to 30°C within 48 to 96 hours. With a more preferable medium, the maximal yield can be attained at 25°C within 48 hours. A synthetic or natural medium comprising the following can be used, for example: as a carbon source, a saccharide, such as glucose, sucrose, starch, dextrin, brown sugar lump, wheat bran, or rice bran; as a nitrogen source, an ammonium salt (e.g., ammonium sulfate, ammonium chloride, or ammonium nitrate) and an inorganic nitrogen source, such as nitrate, or an organic nitrogen source, such as yeast extract, corn steep liquor, meat extract, wheat germ, polypeptone, cane trash, brewer grain, soybean meal, rice bran, or fish meal; and as an inorganic salt, a salt comprising phosphorus, potassium, manganese, magnesium, or iron, such as monopotassium phosphate, magnesium sulfate, manganese sulfate, or ferrous sulfate. When shake culture is performed, an additive such as an antifoaming agent may be added, according to need. Since bacteria of the genus *Bacillus* are aerobic, culture is preferably carried out under aerobic conditions, and solid culture or liquid culture, such as aeration agitation culture or shake culture, is preferable. Culture is carried out preferably at 10°C to 50°C, and more preferably at 15°C to 40°C, at a pH level of preferably 5 to 9, and more preferably of 6 to 8. As described in Example 5, a mixed medium comprising bean curd refuse, wheat bran, and rice bran is preferably used as a culture medium that can satisfy the aforementioned requirements. Since these substances are forms of industrial waste, culture using a medium comprising bean curd refuse, wheat bran, and rice bran is preferable from the viewpoint of effective use of industrial waste.

According to the technique of plant disease control of the present invention, the aforementioned culture solution containing BAL bacteria can be used without any processing. In order to enhance the effect of disease control, a culture solution may be subjected to membrane separation, centrifugation, separation by filtration, or other techniques to obtain a solution with a higher concentration, and the resulting concentrate can be preferably used.

In the present invention, a dehydration product of the aforementioned culture solution containing BAL bacteria can be used. Also, the aforementioned culture solution containing BAL bacteria may be centrifuged, the obtained supernatant may be dehydrated, and the product can then be used. Further, the sedimented bacteria resulting from the centrifugation of the culture solution containing BAL bacteria may be washed with water and dehydrated, and the dehydration product thereof may be used (approximately 50% to 100% by weight of the dehydration product is usually bacteria). The culture solution containing BAL bacteria is allowed to adsorb to a porous adsorbent material, such as powdered activated carbon, diatomaceous earth, or talc, the adsorbent material is dehydrated, and the resultant (which usually comprises $1 \times 10^8$ to $10^9$ cfu/g of bacteria) may also be used. In either case, dehydration may be carried out by conventional techniques, such as lyophilization or drying under reduced pressure. The dehydration products may further be ground by a means such as a ball mill, following dehydration. A specific example of a method for preparing the dehydration product using BAL bacteria is described. To a 300-ml triangular flask containing 100 ml of AG medium (1% glucose (Wako Pure Chemical Industries, Ltd.), 1% polypeptone (Nihon Pharmaceutical Co., Ltd.), 0.1% $KH_2PO_4$ (Wako Pure Chemical Industries, Ltd.), and 0.05% $MgSO_4 \cdot 7H_2O$ (Wako Pure Chemical Industries, Ltd.), pH 7.00, high-pressure sterilization for 15 minutes), a platinum loopful of a slant culture product of BAL bacteria is introduced, and shake culture is conducted at 25°C for 24 hours (120 rpm). Subsequently, the obtained culture product (100 $\mu$l) is introduced into 100 ml of the AG medium, and culture is conducted at 25°C for 48 hours under aerobic conditions. The culture solution is centrifuged to separate the culture supernatant from the culture sediment, the supernatant is recovered, and the sediment is washed with water to obtain bacteria. Alternatively, this culture product is applied dropwise and allowed to adsorb to a porous adsorbent material, such as powdered activated carbon (Wako Pure Chemical Industries, Ltd.), diatomaceous earth (Wako Pure Chemical Industries, Ltd.), or talc (Wako Pure Chemical Industries, Ltd.), which fills a glass tube or a transparent plastic tube with a diameter of 1.8 cm and a length of 10 to 15 cm, to obtain bacteria. The thus obtained culture supernatant, washed bacteria, or adsorbed bacteria are dehydrated via lyophilization or drying under reduced pressure and are then ground by a ball mill. Thus, a dehydration product containing BAL bacteria can be obtained.

In the present invention, BAL bacteria can be used in the form of a culture solution, concentrate, or dehydration product alone. BAL bacteria may be optionally combined with other arbitrary components to be prepared in a form similar to a general microbial preparation (e.g., a powder, wettable powder, emulsion, solution, flowable agent, or embrocation). Examples of other arbitrary components to be combined with BAL bacteria include a solid vehicle and an adjuvant. Examples of a solid vehicle include a powder of organic matter, such as bentonite, diatomaceous earth, a talc compound, pearlite, vermiculite, carboxymethylcellulose sodium (CMC), brewer grain, cane trash, bean curd refuse, wheat bran, chitin, rice bran, and wheat flour. Examples of an adjuvant include a fixing agent and a thickener, such as gelatin, gum arabic, saccharides, and gellan gum. Brewer grain, cane trash, bean curd refuse, wheat bran, and rice bran are all forms of industrial waste, and thus, use thereof in the present invention is preferable from the viewpoint of effective utilization of industrial waste. An example of the control agent or material of the present invention, which comprises a culture solution containing BAL bacteria in combination with industrial waste, such as bean curd refuse, is described in detail. To a 300-ml triangular flask comprising 100 ml of AG medium (1% glucose (Wako Pure Chemical Industries, Ltd.), 1% polypeptone (Nihon Pharmaceutical Co., Ltd.), 0.1% $KH_2PO_4$ (Wako Pure Chemical Industries, Ltd.), and 0.05% $MgSO_4 \cdot 7H_2O$ (Wako Pure Chemical Industries, Ltd.), pH 7.00, high-pressure sterilization for 15 minutes), a platinum loopful of a slant culture product of BAL bacteria is introduced, reciprocal shake culture is conducted at 25°C (120 rpm), and the concentration of BAL bacteria is adjusted to $1 \times 10^7$ cfu/ml. Separately, 1,300 g of bean curd refuse, 600 g of wheat bran, and 100 g of rice bran are mixed (hereafter, this mixture is referred to as a "basic growth medium"). The aforementioned culture solution (1 ml) is introduced into 100 ml of basic growth medium, and stationary culture is then conducted for 72 to 120 hours with thorough agitation once a day in the dark. Following stationary culture, the culture product is dehydrated, and the control agent or material of the present invention is obtained. The thus obtained control agent or material contains BAL bacteria in an amount of approximately $4 \times 10^{9-10}$ cfu per g thereof.

As described above, BAL bacteria are isolated from a residue of edible mushroom culture. Accordingly, the residue of edible mushroom culture containing BAL bacteria can also be used as the control agent or material of the present invention. The residue of edible mushroom culture may be dehydrated and ground, according to need. When the bacteria concentration in the residue of edible mushroom culture or a dehydration product thereof is low, the culture solution, concentrate, or dehydration product of BAL bacteria may be added thereto, according to need. In such a case, it is preferable that such a substance be added to bring the final BAL bacteria concentration in the residue to $1 \times 10^{9-10}$ cfu/ml or higher.

BAL bacteria that have been propagated in industrial waste can also be used as the control agent or material of the present invention. Examples of such control agent or material include BAL bacteria that have been propagated in wheat bran, rice straw, wheat straw, corn stover, or used mushroom bed (a residue of mushroom culture, such as a residue of edible mushroom culture). Such an embodiment of the present invention is preferable from the viewpoint of effective utilization of industrial waste. The BAL bacteria concentration in the thus-obtained control agent or material is generally $1 \times 10^7$ to $10^8$cfu/g, although it varies depending on the material to be incorporated. The CN ratio in usable industrial waste is 70% in the case of rice straw, 70% to 90% in the case of wheat straw, and 80% to 90% in the case of corn stover.

A specific example of the aforementioned embodiment of the present invention is described. Equal amounts of wheat bran, wheat straw (5 to 10 cm cut), and water are mixed and agitated, rice bran (nitrogen content: 1.7% to 2.0%) is added in amounts of 5 to 10 kg, and preferably of 5 to 6 kg, based on 100 kg of the mixture. The mixture is loaded indoors where there is no rainfall in the same manner as in the case of composting. Six to seven days after the generation of fermentation heat, 500 ml of BAL bacteria solution (usually containing $1 \times 10^7$ to $10^9$ cfu/ml of bacteria) that has been cultured in AG medium is homogenously diffused, and the solution is agitated, followed by further sedimentation. Thereafter, shoveling is performed 5 or 6 times every 4 to 6 days, and preferably every 4 or 5 days. Upon completion of fermentation, the fermentation product is dehydrated, and a control material containing BAL bacteria is obtained. The completed control material exhibits variable fertilizer components and analyzed property values in accordance with materials to be incorporated. In general, pH is 7.0 to 7.5, the moisture content is 35% to 40%, the N content is 2.5% to 3.0%, the $P_2O$ content is 3.5% to 4.0%, the $K_2O$ content is 2.0% to 2.5%, the CaO content is 1.0%, the MgO content is 1.0% to 1.5%, the organic matter content is 50% to 60%, and the C/N ratio is 0.7 to 1.3. When the control material of the present invention is used, a simple solar method may also be concurrently employed. (With the utilization of solar energy, the land surface is covered by a transparent mulching film to adjust the internal temperature to 30°C to 40°C at 15 cm below the surface and the land surface temperature to 35°C to 40°C in daylight, and treatment is carried out with soil water content of 70% to 80% for 7 to 10 days.) The control material of the present invention is particularly effective for inhibiting invasion or infection of hosts by soil-borne bacteria, fungi, or viruses. Since the control material of the present invention can effectively control soil-borne viruses in soil (see Example 25), it serves as a particularly useful alternative to a methyl bromide soil fumigant.

The methods of applying BAL bacteria to plants are adequately selected in accordance with various conditions, such as the type of plant disease to be controlled, the condition of plant disease development, the plant variety to be treated, or the form of BAL bacteria. For example, application may be carried out by various techniques, such as ground application, indoor application, soil incorporation, soil perfusion, or surface treatment (e.g., seed dressing or seed coating). Thus, the control agent or material of the present invention can be used for the control of seed transmission, soil transmission, or air or water transmission (infection through wind or water). (Techniques for controlling bacterial plant diseases are roughly classified into these 3 categories, in general). More specifically, application is preferably conducted by at least one technique selected from the group consisting of coating plant seeds with various forms of BAL bacteria, perfusion of such bacteria in soil for plant cultivation, incorporation thereof into soil for plant cultivation, application thereof to plant stem or foliage, and contact thereof with a damaged part of a plant. Coating plant seeds with the BAL bacteria can be carried out by, for example, coating plant seeds with a suspension of BAL bacteria in a solution containing a film-forming agent, such as gellan gum, agar, or carboxymethylcellulose sodium, followed by dehydration. Alternatively, plant seeds may be soaked in a similar suspension, followed by dehydration.

When applying the BAL bacteria to plants, other general active ingredients, for example, insecticides, nematicides, miticides, herbicides, fungicides, bactericides, antiviral agents, fertilizers, or soil conditioners (e.g., peat), may be further mixed in, according to need. These substances can also be applied alternately or simultaneously without mixing. In most cases, activities of BAL bacteria used in the present invention are not interfered with by other active ingredients that are concurrently applied, as described in the Examples.

In the present invention, the amount of BAL bacteria applied to plants is adequately determined in accordance with various conditions, such as the type of plant disease to be controlled, the condition of plant disease development, the plant variety to be treated, or the form of the BAL bacteria. When a BAL bacteria-containing solution is applied to the aerial part of a plant infected with black rot, for example, the viable BAL bacteria concentration in the agent is generally approximately $1 \times 10^{7-10}$ cfu/ml, and preferably approximately $1 \times 10^{8-9}$ cfu/ml. The amount of the agent applied is preferably 100 to 250 1/10a. When a dehydrated powder containing BAL bacteria is incorporated into soil contaminated with *Xanthomonas campestris pv. campestris,* the viable BAL bacteria concentration in the dehydrated powder is generally $1 \times 10^{8-10}$ cfu/g, and preferably $1 \times 10^{9}$ cfu/g. The amount of the dehydrated powder applied is preferably 500 to 2000kg/10a, and more preferably 500 to 1000 kg/10a. The control agent or material comprising BAL bacteria is applied to the soil contaminated with *Xanthomonas campestris pv. campestris* in the amount mentioned above and incorporated into the soil via thorough agitation. The soil is then left untreated with soil water content of approximately 30% to 40% at a soil temperature of 20°C to 30°C for 5 to 7 days with careful attention to keep the soil from drying. If such procedure is implemented, seeds of *Brassicaceae* plants will not be infected with black rot, even if they have been sowed in such contaminated soil.

Reference Example: Examination of taxonomic group of DAIJU-SIID2550

The DAIJU-SIID2550 strain was introduced into CM3 Agar (Oxoid, UK), and culture was conducted at 30°C for 2 days. Thereafter, this bacterial strain was used as a test bacterial strain for DNA extraction. Genomic DNA was extracted by the PrepMan method (Applied Biosystems, U.S.A.). The extracted genomic DNA was used as a template, and a 1500- to 1600-bp region of the total nucleotide sequence of the 16S Ribosomal RNA gene (16S rDNA) was amplified by PCR. Thereafter, the amplified 16S rDNA was subjected to sequencing to obtain the 16S rDNA nucleotide sequence of the test strain. The PCR product was purified and subjected to cycle sequencing with the use of the MicroSeqFull 16S rDNA Bacterial Sequencing Kit (Applied Biosystems, U.S.A.). The GeneAmp PCR System 9600 thermal cycler (Applied Biosystems, U.S.A.) was used, the ABI PRISM 3100 DNA Sequencer (Applied Biosystems, U.S.A.) was used, and the obtained nucleotide sequence fragments were assembled with the use of the AutoAssembler 2.1 (Applied Biosystems, U.S.A.). Basic operations from genomic DNA extraction to cycle sequencing were performed in accordance with the protocol of Applied Biosystems, P/N4308132Rev.A.

The nucleotide sequence (SEQ ID NO: 1) of the obtained 16S rDNA was used to conduct a homology search, and the top 10 strains exhibiting high homology were determined. Further, the determined top 10 strains and 16S rDNA of the test strain were used to prepare a molecular phylogenetic tree by the neighbor-joining method, and the related species and the taxonomic group of the test strain were examined. A homology search and phylogenetic tree preparation were carried out with the use of the MicroSeq Mirobial Identification System Software V.1. 4.1. In the homology search, a database, MicroSeq Bacterial Full Gene Library v. 0001 (Applied Biosystems, U.S.A.), was used.

When no strain exhibiting 100% homology was found in the homology search through the MicroSeq Bacterial Full Gene Library, a BLAST homology search was conducted using the DNA nucleotide sequence database, GenBank/DDBJ/EMBL.

As a result of analysis through the MicroSeq Bacterial Full Gene Library, the differences from *Bacillus amyloliquefaciens, B. subtilis subsp. subtilis,* and *B. mojavensis* were found to be 0.45%, 0.65%, and 0.71%, respectively. No bacterial strain exhibiting complete identity was found. According to the molecular phylogenetic tree, 16S rDNA of the strain of interest was found to be clustered with and related to *Bacillus amyloliquefaciens.* As a result of a BLAST homology search using GenBank/DDBJ/EMBL, the test strain was found to exhibit the highest homology of 99.7% with the *Bacillus sp. Bch* (AF411118) strain, although no strain exhibiting complete identity was found.

Accordingly, the DAIJU-SIID2550 (accession number: FERM BP-10114) strain was found to be a novel bacterial strain of the genus *Bacillus.*

Hereafter, examples concerning plant disease control with the use of BAL bacteria are described.

Example 1

Determination of timing of maximal growth of BAL bacteria

The species related to *Bacillus amyloliquefaciens* (a platinum loopful) deposited as the DAIJU-SIID2550 strain at the International Patent Organism Depositary of the National Institute of Advanced Industrial Science and Technology (accession number: FERM BP-10114) (hereafter referred to as "BAL bacteria") was suspended in 9 ml of sterilized water, 100 μl of the resulting suspension was added to 100 ml of AG medium (1% glucose (Wako Pure Chemical Industries, Ltd.), 1% polypeptone (Nihon Pharmaceutical Co., Ltd.), 0.1% $KH_2PO_4$ (Wako Pure Chemical Industries, Ltd.), and 0.05% $MgSO_4 \cdot 7H_2O$ (Wako Pure Chemical Industries, Ltd.), pH 7.00, high-pressure sterilization for 15 minutes) in a 300-ml triangular flask or 100 ml of a potato decoction liquid medium with 2% sucrose. (The potato decoction was prepared by cutting 200 g of potato into about 1 cm cubes, adding approximately 1 liter of distilled water, and boiling the resultant at low heat for 30 to 40 minutes, followed by filtration through two-ply gauze. 2% sucrose was added to the filtrate, and distilled water was added to bring the amount to 1 liter. The resultant is referred to as "PS medium" in the heat chamber 1.) Shake culture was carried out using a reciprocal shaker with an amplitude of 10 cm at 120 rpm at 25°C for 144 hours. 0 hours, 24 hours, 48 hours, 72 hours, and 144 hours after the initiation of culture, 1 ml of culture solution was sampled. The sampled culture solution was added to 9 ml of sterilized water, and the mixture was thoroughly mixed to prepare a 10-fold diluent. Thereafter, 1 ml of the sample solution was successively sampled and added to 9 ml of sterilized water. This procedure was repeated to prepare $10^{7\text{-}8}$-fold diluents (10-serial dilution). Each of the diluents was dispensed into a YPA plate (comprising peptone, yeast, and salt) in amounts of 100 μl in a 9-cm petri dish, uniformly coated with the use of a Conradi stick, and cultured at 25°C for 72 hours, following which the number of colonies formed was determined. Thus, the number of BAL bacteria (relative value) at each time point was obtained (Table 1). The timing of maximal growth was 48 or 72 hours after the initiation. This indicates that the maximal control effects can be attained 48 to 72 hours after the initiation of culture, when culture is conducted in the presence of BAL bacteria and *Xanthomonas campestris pv. campestris.*

Table 1

| Culture duration (hours) | 0 | 24 | 48 | 72 | 144 |
|---|---|---|---|---|---|
| AG medium | 1 | 5,000 | 85,000 | 74,000 | 63,000 |
| PS medium | | - | 74,000 | 124,000 | 115,000 |

* Cell counts were represented by relative values at each time point when the cell count at the initiation of culture was determined to be 1 (cfu/ml).

Example 2

Inhibition of *Xanthomonas campestris pv. campestris* proliferation via BAL bacteria

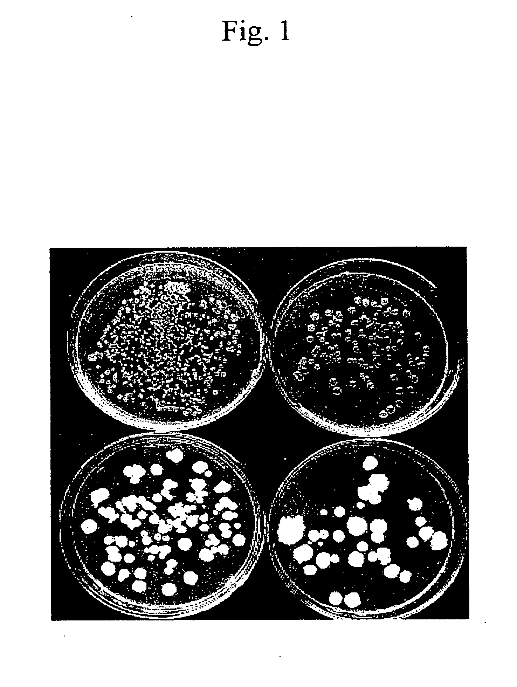

A loopful of BAL bacteria was scraped off from YPA slant medium and introduced into 100 ml of AG medium contained in a 300-ml triangular flask. The resultant was then subjected to shake culture using a reciprocal shaker with an amplitude of 10 cm at 120 rpm at 25°C for 48 hours. A suspension of *Xanthomonas campestris pv. campestris* (100 μl) was introduced into this culture solution and shake culture was conducted for an additional 48 hours under the same conditions. The suspension of *Xanthomonas campestris pv. campestris* used herein was prepared by scraping a loopful of *Xanthomonas campestris pv. campestris* cultured in a potato decoction agar medium with 2% sucrose (hereafter abbreviated to "PSA") slant medium and suspending the same in 9 ml of sterilized water. As a comparative experiment (a control group), 100 μl of the suspension of *Xanthomonas campestris pv. campestris* was introduced into 100 ml of AG medium into which BAL bacteria had not been introduced, and shake culture was similarly carried out at 25°C for 48 hours. Each of the culture solutions was diluted by a serial dilution technique (see Example 1), 100 μl each of the diluents (100,000-fold, 1,000,000-fold, and 10,000,000-fold diluents) was applied to a YPA plate (diameter: 9 cm), culture was conducted at 25°C for 72 hours, and the colony count for the generated *Xanthomonas campestris pv. campestris* was determined. Fig. 1 shows the appearance of the colony 72 hours after the initiation of culture (left: 1,000,000-fold dilution; right: 10,000,000-fold dilution). In Fig. 1, the upper part represents the culture product of *Xanthomonas campestris pv. campestris* alone, and the lower part represents the culture product of *Xanthomonas campestris pv. campestris* together with BAL bacteria. Table. 2 shows the results of the determination of the colony count for *Xanthomonas campestris pv. campestris.* When *Xanthomonas campestris pv. campestris* was subjected to shake culture together with BAL bacteria at 25°C for 48 hours, proliferation of *Xanthomonas campestris pv. campestris* was completely inhibited.

Table 2

| Colony count for *Xanthomonas campestris pv. campestris* | | |
|---|---|---|
| Dilution factor | With the use of BAL bacteria-containing AG medium | With the use of AG medium |
| 100,000-fold | 0 | 744 |
| 1,000,000-fold | 0 | 136 |
| 10,000,000-fold | 0 | 48 |

Example 3

Preparation of seeds artificially contaminated with *Xanthomonas campestris pv. campestris*

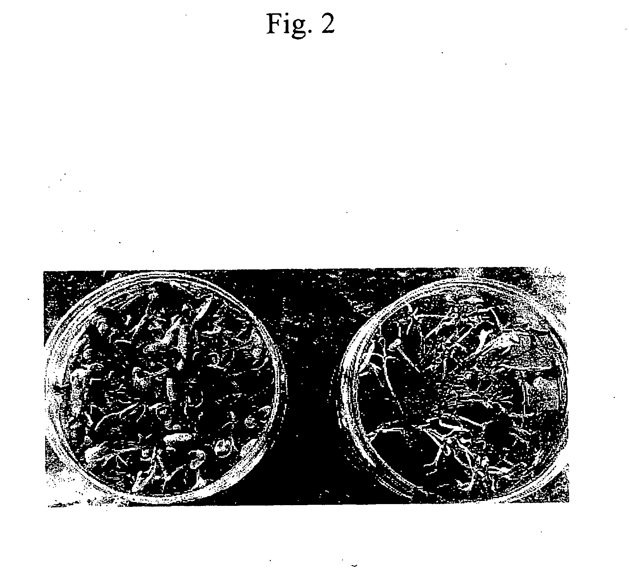

Bok choy seeds (34 g, variety: Fuyushoumi) were wrapped in gauze and soaked in 500 ml of a 150-fold diluted solution of Chemicron G (neutral calcium hypochlorite; available chlorine: 70%) for 10 minutes in order to disinfect the seeds. After the seeds were disinfected, they were washed under running water for 1 hour, and moisture was removed therefrom, followed by dehydration at 35°C overnight. Separately, *Xanthomonas campestris pv. campestris* was previously cultured on 5 PSA plates for 3 days, and all the grown bacteria were scraped off and suspended in 100 ml of distilled water to prepare a $1 \times 10^{12}$cfu/ ml *Xanthomonas campestris pv. campestris* solution. The disinfected seeds (17 g) were soaked in the *Xanthomonas campestris pv. campestris* solution for 20 minutes and then dehydrated at 35°C overnight to prepare artificially contaminated seeds. As a result of inspection of the degree of contamination thereof, the number of contaminating bacteria was found to be $10^{5-6}$ cfu per contaminated seed, and the percentage of the pretreated seeds contaminated was 100%. When the contaminated seeds were introduced into sterilized soil, lesions were frequently observed in cotyledons (Fig. 2). In Fig. 2, the left half shows the results of application of control seeds (a comparative experiment), and the right half shows the results of introduction of seeds artificially contaminated with *Xanthomonas campestris pv. campestris.*

Example 4

Effects of coating seeds artificially contaminated with *Xanthomonas campestris pv. campestris* with BAL bacteria

Seeds artificially contaminated with *Xanthomonas campestris pv. campestris* were treated in the following manner. BAL bacteria, which had been previously cultured on 2 YPA plates, were suspended in 1) 50 ml of an aqueous solution of 0.5% gellan gum (Scott Laboratories, Inc.) or 2) 50 ml of an aqueous solution of 1.5% agar (Wako Pure Chemical Industries, Ltd.), and the solution was stirred with a magnetic stirrer to homogenously suspend BAL bacteria. To such 50 ml of suspension, 300 mg of seeds artificially contaminated with *Xanthomonas campestris pv. campestris* obtained in Example 3 were soaked, allowed to stand overnight, and removed therefrom, followed by air drying. As a comparative experiment (a control group), seeds artificially contaminated with *Xanthomonas campestris pv. campestris* were similarly soaked in 50 ml of an aqueous solution of 0.5% gellan gum or 50 ml of an aqueous solution of 1.5% agar containing no BAL bacteria, followed by air drying. The air-dried seeds were introduced onto YPA plates, culture was conducted at 25°C for 72 hours, and whether or not X*anthomonas campestris pv. campestris* was grown in the vicinity of the seeds was investigated. Based on the results of investigation, the damage imposed on each group was calculated by the following equation.

Damage = the number or seeds that allowed the growth of *Xanthomonas campestris pv. campestris* in the vicinity thereof / the total number of introduced seeds

Based on the calculated damage, the control titer was calculated by the following equation.

Control titer = 100 – (damage of the treatment group / damage of the control group) x 100

The results are shown in Table 3. When seeds artificially contaminated with *Xanthomonas campestris pv. campestris* were coated with BAL bacteria, proliferation of *Xanthomonas campestris pv. campestris* adhered to the seeds was inhibited. This indicates that BAL bacteria can disinfect seeds contaminated with *Xanthomonas campestris pv. campestris.*

Table 3

| | Control titer |
|---|---|
| 1) Coating with an aqueous solution of gellan gum having BAL bacteria suspended therein | 100 |
| 2) Coating with an aqueous solution of agar having BAL bacteria suspended therein | 100 |

Example 5

BAL bacteria culture using bean curd refuse medium

To 100 ml of bean curd refuse that had been sterilized at 105°C for 20 minutes, 1 ml of culture solution of $1 \times 10^7$ cfu/ml of BAL bacteria was mixed, and culture was conducted in the dark at 25°C for 48 hours to obtain a culture product of BAL bacteria in bean curd refuse. The obtained culture product of BAL bacteria in bean curd refuse (500 mg) was suspended in 9 ml of sterilized water, 100 μl each of the diluents obtained by 10-serial dilution in the same manner as in Example 1 was uniformly applied to a YPA plate in a 9-cm petri dish using a Conradi stick, and the number of the BAL bacteria in the culture product of BAL bacteria in bean curd refuse was determined. A fraction of the culture product of BAL bacteria in bean curd refuse was separated and dehydrated at 100°C overnight, and a moisture content was determined. The number of BAL bacteria in the culture product of BAL bacteria in bean curd refuse was $4.3 \times 10^9$ cfu/g, and the moisture content was 87.6%.

As a result of this experiment, it was confirmed that BAL bacteria be capable of proliferation with the use of industrial waste, i.e., bean curd refuse, as a medium. The product of dry-heat dehydration of the culture product of BAL bacteria in bean curd refuse obtained in this example has adequate bacteria concentration and moisture content. Accordingly, this product was found to be usable as a starter for preparing the control agent or material of the present invention comprising BAL bacteria.

Example 6

Effects of coating seeds artificially contaminated with *Xanthomonas campestris pv. campestris* with powder of culture product of BAL bacteria in bean curd refuse

The culture product of BAL bacteria in bean curd refuse (moisture content: 87%) obtained in Example 5 was dehydrated at 35°C overnight and ground with a ball mill to obtain a powder of culture product of BAL bacteria in bean curd refuse. The powder of culture product of BAL bacteria in bean curd refuse (500 mg each) was added to 1) 50 ml of an aqueous solution of 0.5% carboxymethylcellulose sodium (hereafter abbreviated to CMC, Wako Pure Chemical Industries, Ltd.) or 2) 50 ml of an aqueous solution of 0.5% gellan gum, and the mixture was stirred with a magnetic stirrer for 10 minutes. Seeds artificially contaminated with *Xanthomonas campestris pv. campestris* (300 mg) were wrapped in gauze, soaked in each solution for 10 minutes to allow the BAL bacteria to firmly adhere to the seed surface, and dehydrated at 35°C overnight. Thus, the seeds coated with the powder of culture product of BAL bacteria in bean curd refuse were prepared. As a comparative experiment (a control group), seeds artificially contaminated with *Xanthomonas campestris pv. campestris* were soaked in an aqueous solution of 0.5% CMC or 0.5% gellan gum to which no powder of culture product of BAL bacteria in bean curd refuse had been added, followed by dehydration.

3) The powder of culture product of BAL bacteria in bean curd refuse (2 mg) was mixed with 200 mg of seeds artificially contaminated with *Xanthomonas campestris pv. campestris* and 16 μl of an aqueous solution of 0.5% CMC (seed dressing).

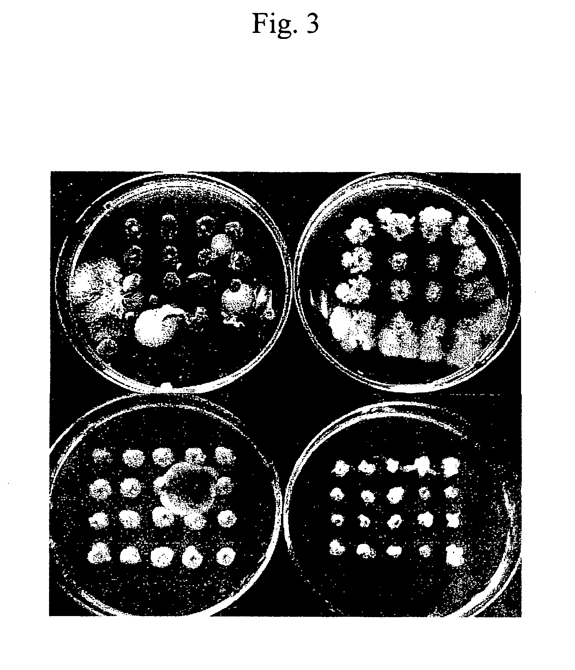

The processed seeds (20 seeds each) were introduced into NSCAA medium (Randhawa, P. S. and Schaad, N. W., 1984, Phytopathology 74: 268-272) and culture was conducted at 25°C for 72 hours. Whether or not *Xanthomonas campestris pv. campestris* would grow in the vicinity of the seeds was inspected. With regard to the above experiments 1) and 2), the appearance of samples after the culture is shown in Fig. 3. In Fig. 3, the upper left portion represents a group treated with an aqueous CMC solution (control group 1)), the upper right portion represents a group treated with an aqueous CMC solution comprising the powder of culture product of BAL bacteria in bean curd refuse (a treatment group 1)), the lower left portion represents a group treated with an aqueous solution of gellan gum (control group 2)), and the lower right portion represents a group treated with an aqueous solution of gellan gum comprising the powder of culture product of BAL bacteria in bean curd refuse (treatment group 2)).

The number of seeds that had allowed *Xanthomonas campestris pv. campestris* to grow in the vicinity thereof was inspected, the "damage" was calculated, and the "control titer" was determined. The "damage" and the "control titer" are as defined in Example 4.

The results are shown in Table 4. In all the experiments 1) to 3), it was confirmed that *Xanthomonas campestris pv. campestris* was controlled. Specifically, coating seeds artificially contaminated with *Xanthomonas campestris pv. campestris* with the powder of culture product of BAL bacteria in bean curd refuse enables the inhibition of proliferation of *Xanthomonas campestris pv. campestris* adhered to the seeds. As an auxiliary substance of coating, CMC was found to be more suitable than gellan gum. This indicates that coating seeds artificially contaminated with *Xanthomonas campestris pv. campestris* with the mixture of the powder of culture product of BAL bacteria in bean curd refuse and the aqueous CMC solution enables the inhibition of contamination of seeds with *Xanthomonas campestris pv. campestris.*

Table 4

| | Control titer |
|---|---|
| 1) Treatment with the powder of culture product of BAL bacteria in bean curd refuse containing the aqueous CMC solution | 84.2 |
| 2) Treatment with the powder of culture product of BAL bacteria in bean curd refuse containing the aqueous solution of gellan gum | 25.0 |
| 3) Treatment with the powder of culture product of BAL bacteria in bean curd refuse (seed dressing) | 60.0 |

## Example 7

### (1) Preparation of residue of edible mushroom culture

The residue of edible mushroom culture was prepared in the following manner.

Cut fresh rice straw was prepared 1 month before the cultivation of edible mushroom. The fresh rice straw was supplemented with soybean cake and rice bran to bring the C/N ratio to around 70% and ricked to adequate width and height. Water was sprayed every 5 days, and shoveling was carried out 4 times until it was decomposed to prepare a compost.

The thus obtained compost was cut to 15 to 18 cm thickness in a cultivation room and used as a mushroom bed. When the temperature of the mushroom bed was decreased to 24°C, a starter *(Agaricus bisporus* 'White mushroom') was introduced thereinto, and edible mushrooms were cultivated under conditions of temperature of 16°C, humidity of 96%, and carbon dioxide of 3,000 ppm. One week after the introduction of the starter, upon elongation of mycelia from the surface of the mushroom bed, the entire mushroom bed was covered with soybean dust, and rotary agitation was performed. Two weeks thereafter, 88 kg of black peat moss was placed per $m^2$ of the bed. Edible mushrooms were harvested 60 days after the introduction of the starter, the used bed was disinfected at 80°C for 30 minutes. The residue of edible mushroom culture was thus obtained. (Agaricus brown mushroom may be used as a starter, and the mushroom may be harvested at an adequate time after the introduction of the starter, e.g., 60 to 90 days thereafter.)

### (2) Test of inhibition of the proliferation of *Xanthomonas campestris pv. campestris* in soil contaminated with *Xanthomonas campestris pv. campestris*

*Xanthomonas campestris pv. campestris* was introduced into sterilized soil to prepare soil contaminated with *Xanthomonas campestris pv. campestris* ($1 \times 10^{12}$ cfu/ml of *Xanthomonas campestris pv. campestris*). The soil contaminated with *Xanthomonas campestris pv. campestris* was allowed to stand at 25°C for 1 day, 50 g of the residue of edible mushroom culture was incorporated relative to 1 liter of the soil contaminated with *Xanthomonas campestris pv. campestris* (i.e., 2,000 kg of the residue per 10 a of the soil), the mixture was allowed to stand at 25°C for 5 days, and the *Xanthomonas campestris pv. campestris* bacteria count in soil was inspected. As a comparative experiment (a control group), soil was contaminated with *Xanthomonas campestris pv. campestris,* and the *Xanthomonas campestris pv. campestris* bacteria count in soil was similarly inspected regarding soil into which the residue of edible mushroom culture had not been incorporated.

The *Xanthomonas campestris pv. campestris* bacteria count in soil was determined in the following manner. That is, 20 g of soil was put through a 300-mesh sieve, the sieved soil was introduced into 80 ml of physiological saline (0.85% NaCl) to obtain a suspension, and the suspension was shaken for 30 minutes using a reciprocal shaker with an amplitude of 10 cm at 120 rpm, followed by centrifugation at 500 rpm for 5 minutes. The supernatant obtained via centrifugation was subjected to 10-series dilution with distilled water containing 0.05% agar, 500 μl of the diluent was applied dropwise to NSCAA medium (Randhawa, P. S. and Schaad, N. W., 1984, Phytopathology 74: 268-272) and uniformly coated thereon using a Conradi stick, culture was conducted at 25°C, and the number of colonies exhibiting a configuration peculiar to *Xanthomonas campestris pv. campestris* was inspected to determine the *Xanthomonas campestris pv. campestris* bacteria count.

The *Xanthomonas campestris pv. campestris* bacteria count per g of dry soil was $1.09 \times 10^7$ cfu when the residue of edible mushroom culture was added (a treatment group) and $7.06 \times 10^7$ cfu when the residue of edible mushroom culture was not added (a control group).

The control titer was calculated by the following equation, which was 84.6%. The results are summarized in Table 5.

Control titer = 100 - (the *Xanthomonas campestris pv. campestris* bacteria count of the treatment group)/(the *Xanthomonas campestris pv. campestris* bacteria count of the control group) × 100

The test results indicate that the proliferation of *Xanthomonas campestris pv. campestris* can be significantly inhibited by incorporating the residue of edible mushroom culture into soil.

Table 5

| | The *Xanthomonas campestris pv. campestris* bacteria count (cfu/g of dry soil) | Control titer(%) |
|---|---|---|
| Treatment group | $1.09 \times 10^7$ | 84.6 |
| Control group | $7.06 \times 10^7$ | - |

Example 8

Effects of soaking seeds artificially contaminated with *Xanthomonas campestris pv. campestris* in a culture solution of BAL bacteria and effects of inhibiting development of black rot upon incorporation of the residue of edible mushroom culture into soil

BAL bacteria were introduced into AG medium, and shake culture was conducted for 7 days to prepare a culture solution of BAL bacteria with a bacteria concentration of $1 \times 10^6$ cfu/ml. Seeds artificially contaminated with *Xanthomonas campestris pv. campestris* (200 mg) were wrapped in gauze and soaked in the culture solution for a given period of time. Soaking was carried out for 10 minutes, 20 minutes, 40 minutes, and 60 minutes. After soaking, moisture was removed from seeds, the seeds were spread, and they were then dehydrated at 35°C overnight. For the purpose of comparison, seeds artificially contaminated with *Xanthomonas campestris pv. campestris* were soaked in AG medium into which no BAL bacteria had been introduced for the same period of time, followed by dehydration.

As the soil into which the seeds that had been treated in the above-described manner were introduced, soil disinfected with the use of a steam soil-disinfecting apparatus (SB-150, Marubun Seisakusyo Co. Ltd.,) at 100°C for 30 minutes (hereafter referred to as "disinfected soil") and mixed soil prepared by incorporating 2,000 kg/10 a of the residue of edible mushroom culture into disinfected soil (see Example 7 (1)) (hereafter referred to as "soil containing the residue of edible mushroom culture") were prepared. The soil (150 ml each) was introduced into sealable containers, and 20 seeds artificially contaminated with *Xanthomonas campestris pv. campestris* processed as described above were introduced, followed by treatment at 25°C.

As a control group, seeds artificially contaminated with *Xanthomonas campestris pv. campestris* were introduced into disinfected soil to prepare a test group.

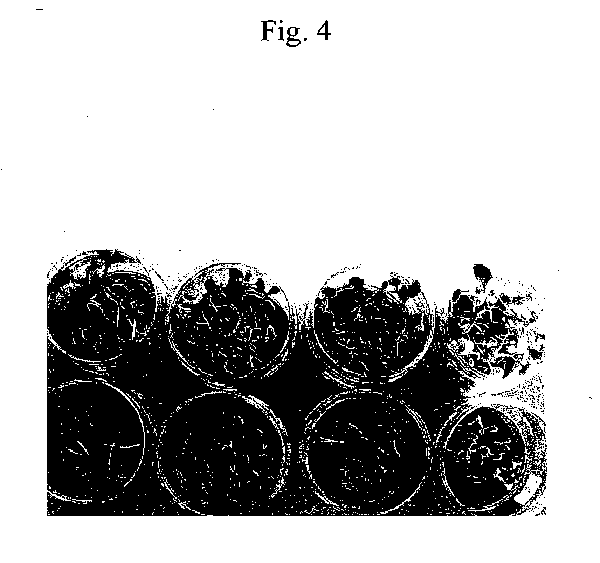

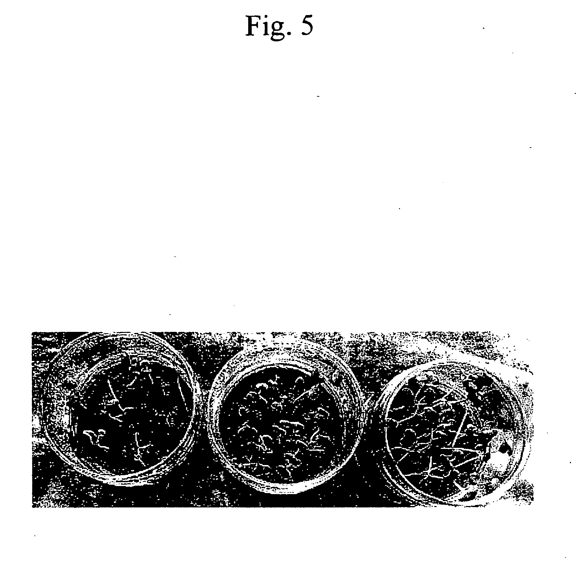

The appearances of samples 9 days after the application are shown in Fig. 4 and in Fig. 5. In Fig. 4, the upper portion represents the results of cultivation under the conditions of "soaking in AG medium containing BAL bacteria and the soil additionally comprising the residue of edible mushroom culture," and the lower portion represents the results of cultivation under the conditions of "soaking in AG medium containing BAL bacteria and the disinfected soil" (the soaking duration is for 10 minutes, 20 minutes, 40 minutes, and 60 minutes from the left). In Fig. 5, the leftmost portion represents the results of cultivation under the conditions of "soaking in AG medium containing no BAL bacteria and disinfected soil," the center portion represents the results of cultivation under the conditions of "soaking in AG medium containing BAL bacteria and disinfected soil," and the rightmost portion represents the results of cultivation under the conditions of "soaking in AG medium containing BAL bacteria and the soil additionally comprising the residue of edible mushroom culture" (the soaking duration is for 60 minutes in all experiments).

The disease symptom of black rot that appeared on the cotyledon 9 days after the application of BAL bacteria was investigated. The investigation was made based on the following 6 criteria of the disease states.

0: no symptom
1: the lesion area accounts for half or smaller of a cotyledon
2: the lesion area accounts for over a cotyledon
3: the lesion area accounts for 1.5 cotyledons
4: the lesion area accounts for 2 cotyledons
5: blighted

Based on the results of investigation, the incidence of disease was calculated by the following equation.

$$\text{Incidence of disease} = (1n_1+2n_2+3n_3+4n_4+5n_5)/(5\times\text{total number of plants investigated})$$

In the equation, $n_1$, $n_2$, $n_3$, $n_4$, and $n_5$ each independently represent the number of individual plants categorized by the criteria 1, 2, 3, 4, and 5.

Based on the determined incidence of disease, the control titer was calculated by the following equation:

$$\text{Control titer} = 100 - \text{incidence in treatment group}/\text{incidence in control group} \times 100$$

The control titers of the above groups are shown in Table 6. As is apparent from Table 6, development of black rot was inhibited by soaking the seeds artificially contaminated with *Xanthomonas campestris pv. campestris* in the culture solution of BAL bacteria. The effects of such inhibition were found to become higher as the soaking duration in the culture solution of BAL bacteria was prolonged. With regard to the test group involving the use of the soil into which the residue of edible mushroom culture was incorporated, the test group with a short duration of soaking in the culture solution of BAL bacteria (e.g., for 10 minutes) produced the control titer substantially the same as the control titer attained when the seeds soaked in the AG medium containing BAL bacteria for 60 minutes were introduced into the disinfected soil.

Table 6

| Control titers of treatment groups | | | | Soil used | |
|---|---|---|---|---|---|
| | | | | Disinfected soil | Soil containing the residue of mushroom culture |
| Soaking liquid used | AG medium containing BAL bacteria | Duration of soaking | 10 minutes | 20.7 | 60.3 |
| | | | 20 minutes | 38.2 | 71.6 |
| | | | 40 minutes | 56.2 | 75.8 |
| | | | 60 minutes | 74.6 | 78.8 |
| | AG medium | Duration of soaking | 10 minutes | 2.7 | 30.1 |
| | | | 20 minutes | -7.5* | 32 |
| | | | 40 minutes | -13.2* | 20.8 |
| | | | 60 minutes | -7.5* | 41.8 |
| * Test groups with negative control titers exhibit larger incidences of diseases than control groups | | | | | |

Example 9

Control of black rot by ground application of a culture solution of BAL bacteria

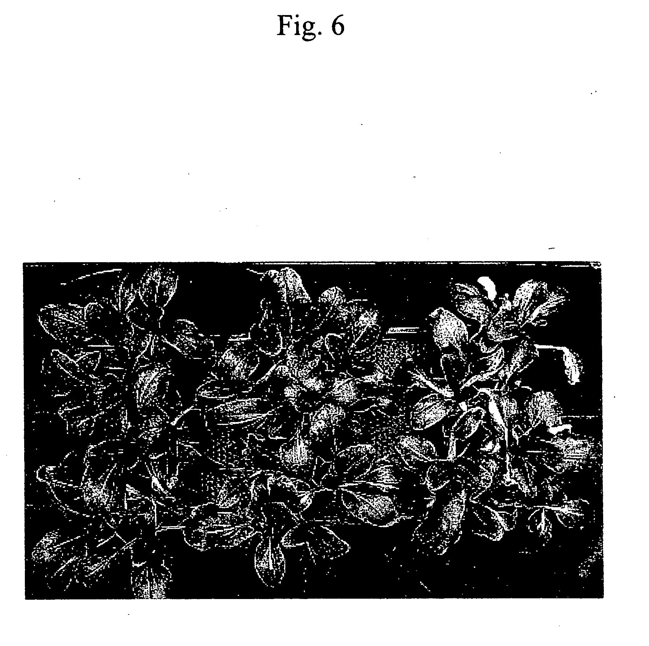

The culture solution of BAL bacteria (300 ml, bacteria concentration: $3 \times 10^8$cfu/ml) that had been shake-cultured in AG medium was sprayed to the leaves of 9 bok choy strains (variety: Fuyushoumi) at the 4-leaf stage using an atomizer. The bok choy strains were allowed to stand for approximately 1 hour, and 300 ml of a solution of *Xanthomonas campestris pv. campestris* was applied thereto using an atomizer. This solution of *Xanthomonas campestris pv. campestris* was prepared by first culturing *Xanthomonas campestris pv. campestris* on 5 PSA plates, scraping all the *Xanthomonas campestris pv. campestris* from the plates, and suspending the same in 150 ml of distilled water to prepare a solution of $10^8$ cfu/ml *Xanthomonas campestris pv. campestris* for introduction. The plants to which the BAL bacteria had been applied were introduced into a moist chamber (humidity: 80%) and kept at 20°C. For the purpose of comparison, a test group to which the culture solution of BAL bacteria was applied by spraying but *Xanthomonas campestris pv. campestris* was not applied and a test group, i.e., 9 bok choy strains to which BAL bacteria were not sprayed but the solution of *Xanthomonas campestris pv. campestris* of the same concentration were sprayed (a control group), were prepared. Fig. 6 shows the aerial part 26 days after the initiation of the experiment. In Fig. 6, the left lane shows the results of cultivation in soil into which "BAL bacteria has been introduced but *Xanthomonas campestris pv. campestris* has not been introduced," the center lane shows the results of cultivation in soil into which "BAL bacteria and *Xanthomonas campestris pv. campestris* have been introduced" (a treatment group), and the right lane shows the results of cultivation in soil into which "BAL bacteria has not been introduced but *Xanthomonas campestris pv. campestris* has been introduced" (a control group). Development of disease was inspected by evaluating the symptom of black rot that appeared on the leave 33 days after the initiation of the experiment. The incidence of the disease on leaves was first determined (the percentage of diseased leaves among the leaves inspected). Subsequently, the control titer was determined by the following equation.

$$\text{Control titer} = 100 - (\text{incidence of the disease on leaves of the treatment group} / \text{incidence of the disease on leaves of the control group}) \times 100$$

The results are shown in Table 7. Spraying of BAL bacteria prior to introduction of *Xanthomonas campestris pv. campestris* resulted in inhibition of black rot development. This indicates that preventive application of a culture solution of BAL bacteria to the aerial part of the *Xanthomonas campestris pv. campestris* host enables inhibition of black rot development and transmission.

Table 7

| | BAL bacteria | *Xanthomonas campestris pv. campestris* | Control titer |
|---|---|---|---|
| Control group | Not introduced | Introduced | - |
| Treatment group | Introduced | Introduced | 58.6 |

Example 10

Effects of conventional fungicides or insecticides on BAL bacteria

Conventional fungicides and insecticides, i.e., Amistar 20 Flowable (Syngenta, azoxystrobin wettable powder), Jimandaisen wettable powder (DSS Hishishou, manzeb wettable powder), Rovral wettable powder (Yashima Kagaku, iprodione wettable powder), Rizolex wettable powder (Sumitomo Chemical Co., Ltd., tolchlofosmethyl wettable powder), Sthana wettable powder (Sumitomo Chemical Co., Ltd., oxolinic acid wettable powder), Daconil 1000 Flowable (SDS, TPN wettable powder), Z-bordeaux (Nihon Nohyaku Co, Ltd., copper wettable powder), Benlate wettable powder (Sumitomo Chemical Co., Ltd., benomyl wettable powder), Ridomyl MZ wettable powder (Syngenta, manzeb/metalaxyl wettable powder), Bestguard water-soluble powder (Sumika Takeda, nitenpyram water-soluble powder), DDVP emulsion (Nihon Nohyaku Co, Ltd., DDVP emulsion), Affirm emulsion (Syngenta, emamectin benzoate emulsion), Admire wettable powder (Bayer CropScience, imidachlopride wettable powder), Lannate 45 wettable powder (Sankyo Agro Co., Ltd., methomyl wettable powder), Padan SG water-soluble powder (Sumika Takeda, cartap water-soluble powder), Mospilan water-soluble powder (Nippon Soda Co., Ltd., acetamiprid water-soluble powder), Elsan emulsion (Nissan Chemical Co. Ltd., PAP emulsion), Actara granule water-soluble powder (Syngenta, Thiamethoxam water-soluble powder), Kotetsu flowable (Nihon Nohyaku Co, Ltd., chlorphenapyr wettable powder), and Torebon emulsion (Mitsui Chemicals, Inc., Etofenprox emulsion), were diluted to given concentrations to prepare chemicals, and filter papers (Toyo Roshi No. 2) were soaked in these chemicals, followed by drying. A suspension of BAL bacteria in distilled water ($2 \times 10^8$ cfu/ml) was then sprayed to the filter papers in amounts of 44 $\mu$l per $cm^2$, the filter papers were dried again, and they were then allowed to stand at 25°C in the dark overnight. The filter papers were cut into 5 to 10 $mm^2$ pieces and culture was conducted on a YPA plate at 25°C in the dark.

The BAL bacteria growing in the vicinity of a piece of 10 $mm^2$ filter paper that had been introduced onto a YPA plate were observed 3 and 4 days after the culture.

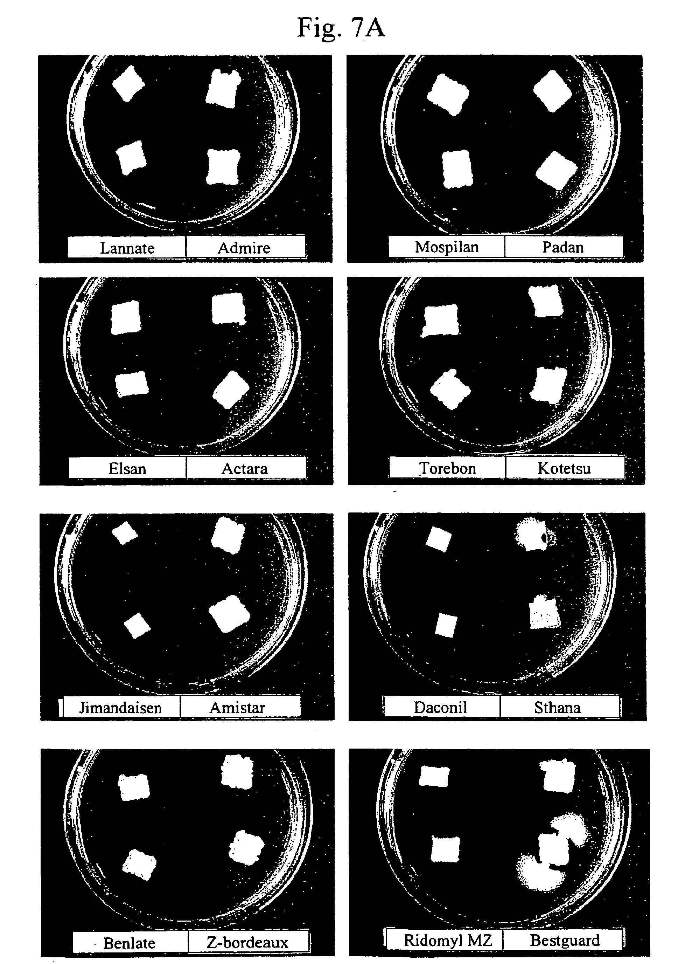

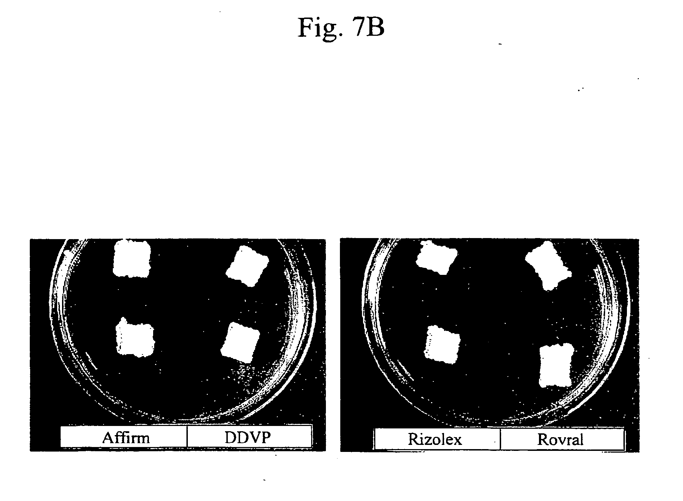

The results are shown in Table 8. Also, the plate 4 days after the culture is shown in Fig. 7. Growth of BAL bacteria was not influenced by fungicides or insecticides except for some chemicals. In the presence of Jimandaisen wettable powders and Ridomyl MZ wettable powders, however, growth of BAL bacteria was very slow, and substantially no colony was observed 3 days after the culture. That is, the growth of BAL bacteria was not observed, but the growth thereof became to be observed gradually from 4 days after the culture and thereafter. In the presence of Sthana wettable powders and the Daconil 1000 flowable agent, the growth of BAL bacteria was not observed at all.

These results indicate that the BAL bacteria-containing control agent or material for plant diseases according to the present invention can be applied in combination with many conventional fungicides or insecticides, or it can be applied alternately or simultaneously without mixing.

Table 8

| Influence of conventional fungicides or insecticides on BAL bacteria | | | |
|---|---|---|---|
| | Agrichemicals | Dilution factors | Condition of bacterial growth* |
| Fungicides | Amistar 20 flowable | 2000 | + |
| | Jimandaisen wettable powder | 1000 | ± |
| | Rovral wettable powder | 1000 | + |
| | Rizolex wettable powder | 1000 | + |
| | Sthana wettable powder | 2000 | - |
| | Daconil 1000 flowable | 1000 | - |
| | Z-Bordeaux - copper wettable powder | 500 | + |
| | Benlate wettable powder | 2000 | + |
| | Ridomyl MZ wettable powder | 1000 | ± |

(continued)

| Influence of conventional fungicides or insecticides on BAL bacteria | | | |
|---|---|---|---|
| | Agrichemicals | Dilution factors | Condition of bacterial growth* |
| Insecticides | Bestguard water-soluble powder | 2000 | + |
| | DDVP emulsion | 2000 | + |
| | Affirm emulsion | 2000 | + |
| | Admire wettable powder | 2000 | + |
| | Lannate 45 wettable powder | 2000 | + |
| | Padan SG water-soluble powder | 1500 | + |
| | Mospilan water-soluble powder | 4000 | + |
| | Elsan emulsion | 2000 | + |
| | Actara granule water-soluble powder | 3000 | + |
| | Kotetsu flowable | 2000 | + |
| | Torebon emulsion | 2000 | + |
| *+: Colony was observed 3 days after the culture; ±: Colony was observed 4 days after the culture due to slow growth; -: No colony was observed. | | | |

Example 11

Inhibition of canker fungi (*Clavibacter michiganensis subsp. michiganensis)* proliferation via BAL bacteria

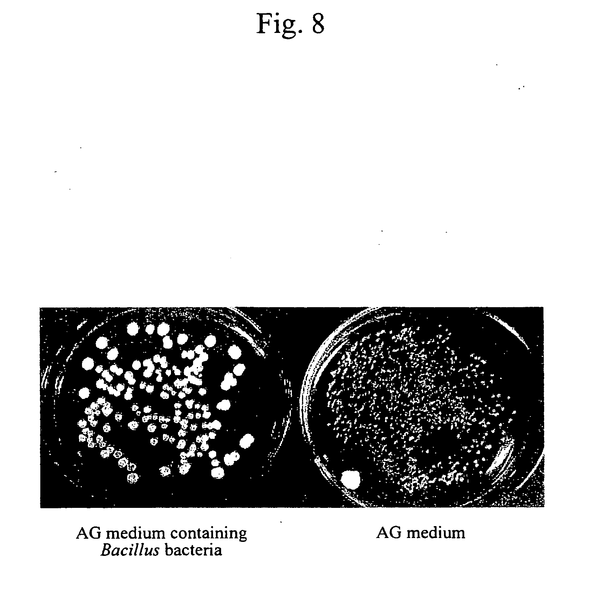

A platinum loopful of BAL bacteria was scraped off from YPA slant medium, introduced into 100 ml of AG medium filled in a 300-ml triangular flask, and then subjected to shake culture using a reciprocal shaker with an amplitude of 10 cm at 120 rpm at 25°C for 48 hours. To such culture solution, 100 μl of a suspension of *Clavibacter michiganensis subsp. michiganensis* was introduced, and shake culture was conducted for an additional 48 hours under the same conditions. The suspension of *Clavibacter michiganensis subsp. michiganensis* used herein was prepared by scraping off a loopful of the *Clavibacter michiganensis subsp. michiganensis* that had been cultured in PSA slant medium, and suspending the same in 9 ml of sterilized water. As a comparative experiment (a control group), 100 μl of a suspension of *Clavibacter michiganensis subsp. michiganensis* was introduced into 100 ml of AG medium into which no BAL bacteria had been introduced, and shake culture was similarly conducted at 25°C for 48 hours. The culture solutions were diluted by serial dilution techniques (see Example 1), 100 μl each of the diluents (100,000-fold, 1,000,000-fold, and 10,000,000-fold diluents) was applied to a 9-cm YPA plate, culture was conducted at 25°C for 72 hours, and the colony count for the grown *Clavibacter michiganensis subsp. michiganensis* was determined. The results of the determination of the colony count are shown in Table 9. The colony appearance is shown in Fig. 8. When *Clavibacter michiganensis subsp. michiganensis* cells were subjected to shake culture at 25°C for 48 hours with BAL bacteria, accordingly, *Clavibacter michiganensis subsp. michiganensis* proliferation was completely inhibited.

Table 9

| Dilution factors | AG medium containing bacteria of the genus *Bacillus* | AG medium |
|---|---|---|
| $10^5$ | 0 | 1079 |
| $10^6$ | 0 | 132 |
| $10^7$ | 0 | 55 |

Example 12

Inhibition of wilt bacteria *(Ralstonia solanacearum)* proliferation via BAL bacteria

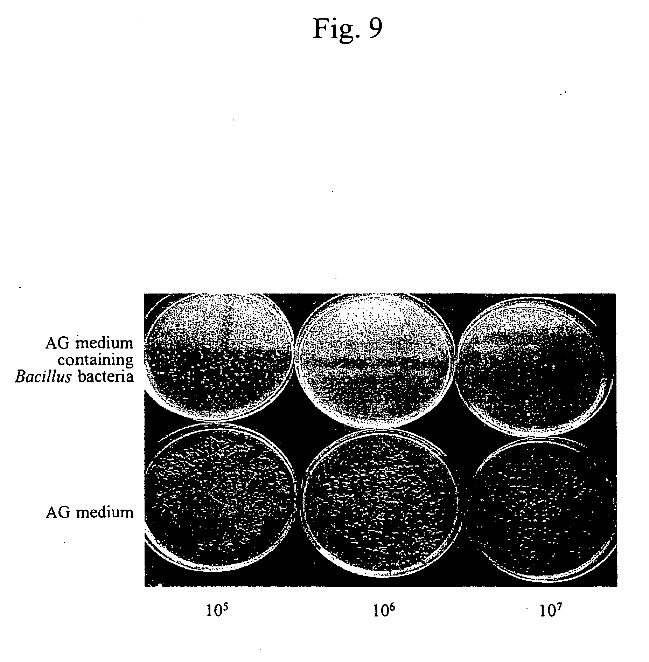

A platinum loopful of BAL bacteria was scraped off from YPA slant medium, introduced into 100 ml of AG medium filled in a 300-ml triangular flask, and shake culture was conducted at 25°C for 48 hours using a reciprocal shaker with an amplitude of 10 cm at 120 rpm. To such culture solution, 100 μl of a suspension of *Ralstonia solanacearum* was introduced, and shake culture was conducted for an additional 120 hours under the same conditions. The suspension of *Ralstonia solanacearum* used herein was prepared by scraping off a loopful of *Ralstonia solanacearum* cultured in PSA slant medium, and suspending the same in 9 ml of sterilized water. As a comparative experiment (a control group), 100 μl of the suspension of *Ralstonia solanacearum* was introduced into 100 ml of AG medium into which no BAL bacteria had been introduced, and shake culture was similarly carried out at 25°C for 120 hours. The culture solutions were diluted by serial dilution techniques (see Example 1), 100 μl each of the diluents (1,000,000-fold and 10,000,000-fold diluents) was applied to a 9-cm YPA plate, culture was conducted at 25°C for 72 hours, and the colony count for the grown *Ralstonia solanacearum* was determined. The results of the determination of the colony count are shown in Table 10. The colony appearance is shown in Fig. 9. When *Ralstonia solanacearum* cells were subjected to shake culture at 25°C for 120 hours with BAL bacteria, accordingly, *Ralstonia solanacearum* proliferation was inhibited.

Table 10

| Dilution factors | AG medium containing bacteria of the genus *Bacillus* | AG medium |
|---|---|---|
| $10^6$ | 48 | 831 |

Example 13

Inhibition of soft rot bacteria *(Erwinia carotovora subsp. carotovora)* proliferation via BAL bacteria

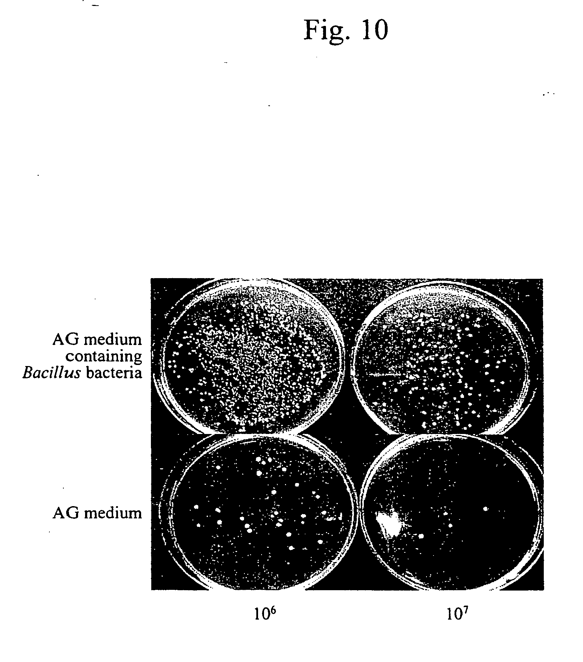

A platinum loopful of BAL bacteria was scraped off from YPA slant medium, introduced into 100 ml of AG medium filled in a 300-ml triangular flask, and subjected to shake culture at 25 °C for 48 hours using a reciprocal shaker with an amplitude of 10 cm at 120 rpm. To such culture solution, 100 μl of a suspension of *Erwinia carotovora subsp. carotovora* was introduced, and shake culture was conducted for an additional 96 hours under the same conditions. The suspension of *Erwinia carotovora subsp. carotovora* used herein was prepared by scraping off a loopful of *Erwinia carotovora subsp. carotovora* cultured in a potato decoction agar medium with 2% sucrose (hereafter abbreviated to "PSA") slant medium, and suspending the same in 9 ml of sterilized water. As a comparative experiment (a control group), 100 μl of the suspension of *Erwinia carotovora subsp. carotovora* was introduced into 100 ml of AG medium into which no BAL bacteria had been introduced, and shake culture was similarly carried out at 25°C for 96 hours. The culture solutions were diluted by serial dilution techniques (see Example 1), 100 μl each of the diluents (1,000,000-fold and 10,000,000-fold diluents) was applied to a 9-cm YPA plate, culture was conducted at 25°C for 72 hours, and the colony count for the grown *Erwinia carotovora subsp. carotovora* was determined. The results of the determination of the colony count are shown in Table 11. The colony appearance is shown in Fig. 10. When *Erwinia carotovora subsp. carotovora* cells were subjected to shake culture at 25°C for 96 hours with BAL bacteria, accordingly, *Erwinia carotovora subsp. carotovora* proliferation was completely inhibited.

Table 11

| Dilution factors | AG medium containing bacteria of the genus *Bacillus* | AG medium |
|---|---|---|
| $10^6$ | 0 | 1259 |
| $10^7$ | 0 | 187 |

Example 14

Inhibition of soft rot of Chinese cabbage

A leaf stalk of Chinese cabbage was washed with tap water, distilled water, and 70% ethanol, in that order, moisture was wiped away using Kimwipes, and the leaf stalk was introduced into a large glass petri dish. Soft rot bacteria, i.e., *Erwinia carotovora subsp. carotovora,* were cultured in YPA slant medium for 3 days, and suspended in 9 ml of distilled water to prepare a $1 \times 10^7$ cfu/ml bacteria solution. To 1 ml of this bacteria solution, 1 ml of culture solution of BAL bacteria was added, the mixture was thoroughly agitated, 8 cotton needles were soaked therein, and the leaf stalk of the Chinese cabbage was scratched with the needles. BAL bacteria were thus introduced. Thereafter, the glass petri dish was sealed with a paraffin film to keep the bacteria from drying, and the petri dish was allowed to stand in an incubator at 30°C overnight. As a comparative experiment (a control group), a mixed solution comprising 1 ml of bacteria solution and 1 ml of distilled water was similarly introduced using needles. Damage evaluation was carried out by determining a piece of the leaf stalk without soft rot as "-" and the leaf stalk with soft rot as "+". The control titer was determined by the following equation.

$$\text{Control titer} = 100 - (\text{Incidence of disease of the treatment group/incidence of disease of the control group}) \times 100$$

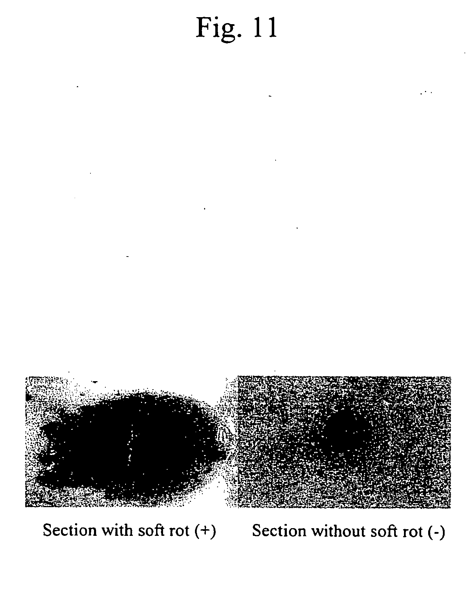

The results are shown in Table 12. The colony appearance 1 day after the initiation of the experiment is shown in Fig. 11.

Table 12

| | Group 1 | Group 2 | Percentage of damage | Control titer |
|---|---|---|---|---|
| Control | + | + | 100 | |
| BAL bacteria mixed | - | - | 0 | 100 |

Example 15

Inhibition of seed transmission with *Burkholderia plantarii* and *Burkholderia glumae*

A culture solution for preparing artificially contaminated rice seeds was prepared by subjecting *Burkholderia plantarii* (the stock strain of the Japan Plant Protection Association) to shake culture in PPG medium (200 g of potato, 3 g of dibasic sodium phosphate dodecahydrate (Wako), 0.5 g of dibasic potassium phosphate (Wako), 5 g of peptone (Nihon Pharmaceutical Co., Ltd.), 3 g of sodium chloride (Wako), 5 g of glucose (Wako), and 1 liter of distilled water) at 25°C for 3 days. Rice seeds to be contaminated were first disinfected by soaking in a 250-fold solution of Chemicron G (Nippon Soda Co., Ltd.) for 30 minutes and then washed with running water for 45 minutes, followed by drying. A culture solution (30 ml) containing 15 g of seeds and the aforementioned bacteria was introduced into a 100-ml beaker to infect the seeds under a reduced pressure with the use of a vacuum pump (Yamato MINIVAC PS-22).

The contaminated seeds (3 g) and 30 ml of culture solution containing BAL bacteria that had been shake-cultured for 5 days using a bean curd refuse extract (50 g of bean curd refuse was suspended in 100 ml of distilled water, filtered through gauze, and high-pressure sterilized at 121 °C for 20 minutes) (hereafter referred to as a "culture solution of BAL bacteria") were introduced into a 50-ml Falcon tube for soaking. The seeds (1.5 g) that had been subjected to soaking at room temperature for 48 hours were introduced into an ice cream cup (made of polyethylene, a diameter of 10 cm, a height of 5.5 cm) containing 100 ml of Kumiai-Ryujyou-Baido D (Kureha Chemical Industry Co., Ltd.). As a control, contaminated seeds that were not soaked in a culture solution of BAL bacteria were similarly introduced. The seeds were forced to germinate in the dark at 32°C, greening and supervision were continued in an artificial climate chamber at 20°C, the entire strains were pulled out 16 days after the application, and disease development was inspected (*Sakumotsu byougenkin kenkyuu gihou no kiso - bunri/baiyo/sesshu (Basics of crop pathogen research techniques - separation/culture/introduction),* Kanichi Ohata (ed.), the Japan Plant Protection Association). Blighted seedlings and browned seedlings were inspected as diseased strains. The incidence of the disease was calculated and the control titer was determined by the following equation.

$$\text{Control titer} = 100 - (\text{incidence of disease of the treatment group/incidence of disease of the control group}) \times 100$$

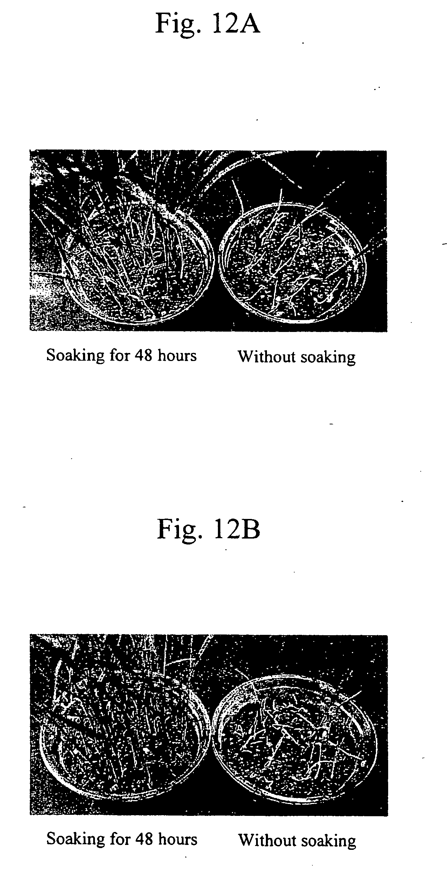

The results are shown in Table 13. The colony appearance 16 days after the initiation of the experiment is shown in Fig. 12A.

Table 13

| Effects of soaking seeds contaminated with *Burkholderia glumae* in culture solution of BAL bacteria | | | | |
|---|---|---|---|---|
| | Number of healthy strains | Number of diseased strains | Incidence of disease | Control titer |
| Contaminated seeds | 1 | 49 | 98.0 | - |
| Soaked seeds | 30 | 15 | 33.3 | 66.0 |

The experiment was also carried out with regard to *Burkholderia glumae* (the stock strain of the Japan Plant Protection Association) in the manner as described above. Concerning bacterial grain rot, blighted seedlings and seriously diseased seedlings (with a half plant length or shorter of a healthy plant) were inspected as diseased strains. The results are shown in Table 14. The appearance of samples 16 days after the initiation of the experiment is shown in Fig. 12B.

Table 14

| Effects of soaking seeds contaminated with *Burkholderia plantarii* in culture solution of BAL bacteria | | | | |
|---|---|---|---|---|
| | Number of healthy strains | Number of diseased strains | Incidence of disease | Control titer |
| Contaminated seeds | 4 | 43 | 91.5 | - |
| Soaked seeds | 33 | 12 | 26.7 | 70.9 |

Example 16

Effects of inhibiting rice blast fungi (*Pyricularia grisea*) spore generation

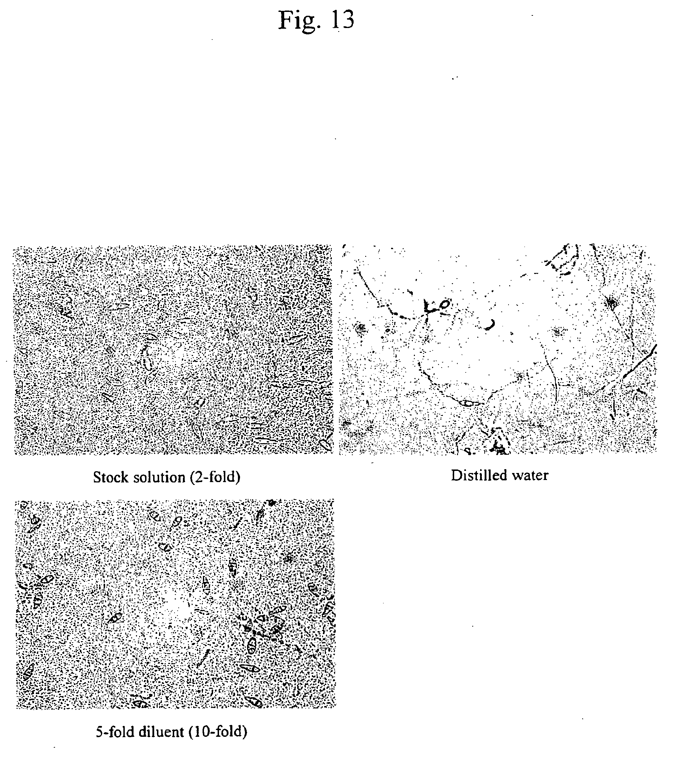

An undiluted culture solution of BAL bacteria, a 5-fold diluted culture solution of BAL bacteria (2-fold or 10-fold as the final concentration at the time of germination testing), and distilled water (a control group) were employed as samples. *Pyricularia grisea* spores (formed by allowing flora to grow in sugar-added oatmeal agar medium and being irradiated with BLB) suspended in PS medium (a medium comprising 2% sugar added to a potato decoction) and 20 μl each of the samples were applied to a hole glass slide, they were thoroughly mixed, the mixture was allowed to stand at 25°C for 24 hours in a moist chamber, and the presence of a germ tube was inspected under a microscope. Fig. 13 shows the *Pyricularia grisea* spores 24 hours after sample mixing. In the stock solution and the 5-fold diluent to which the culture solution was added, germination of *Pyricularia grisea* spores was inhibited, and no germinated spores was observed.

Example 17

Effects of inhibiting rice brown spot fungi *(Cochliobolus miyabeanus)* spore germination

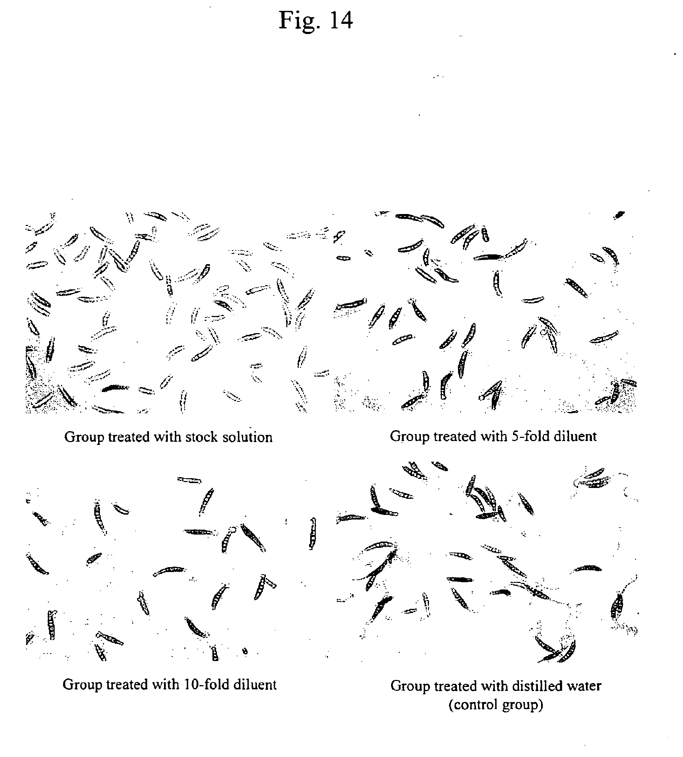

To a 300-ml triangular flask containing 100 ml of YPA medium (0.5% of yeast extract (Nihon Pharmaceutical Co., Ltd.), 1% polypeptone (Nihon Pharmaceutical Co., Ltd.), and 0.5% sodium chloride, pH 7.00, high-pressure sterilization for 20 minutes), a loopful of BAL bacteria cultured in YPA slant medium was introduced, and shake culture was conducted at 25°C for 72 hours (120 rpm). The stock solution, the 5-fold diluent, and the 10-fold diluent of the obtained culture solution were employed as samples. The solution that was similarly treated with distilled water was employed as a control group. *Cochliobolus miyabeanus* spores (prepared by introducing the precultured *Cochliobolus miyabeanus* to PSA medium and culturing the same at 25°C for 7 to 10 days) suspended in distilled water was added in an amount of 100 μl relative to 1 ml of the samples, they were thoroughly mixed, 50 μl of the resultant was introduced onto a hole glass slide, it was then allowed to stand in a moist chamber at 25°C for 24 hours, and the presence or absence and the configuration of the germ tube were observed under a microscope. The *Cochliobolus miyabeanus* spores 24 hours after the sample mixing are shown in Fig. 14. In the stock solution of the culture solution, no spores were germinated. In the 5-fold and 10-fold diluents, germ tube elongation was inhibited, swelling deformation took place, and germ tube elongation was not observed.

Example 18

Control of rice brown spot via ground application of culture solution of BAL bacteria

The culture solution of BAL bacteria or distilled water (as a control group) was applied to rice at 6- to 7-leaf stage (variety: Nihonbare) that had been grown in an artificial climate chamber, the plant was allowed to stand in the bright at 25°C for 16 hours, and a suspension of *Cochliobolus miyabeanus* spores was introduced by spraying 24 hours later. This suspension of spores was prepared by culturing the *Cochliobolus miyabeanus* in PSA medium in advance, pouring distilled water into a culture petri dish, rubbing the flora with a brush to wash away spores, filtering the product through two-ply gauze, and then adjusting the spore concentration to 10 per a 100-fold visual field of the microscope, and adding Tween 20 to result in the concentration of 0.02% therein. Thereafter, the rice plant was covered with a plastic container to prepare a moist chamber at 25°C, and the plant was allowed to stand therein for 24 hours. Six days after the application, the total number of lesions on the fully developed leaves and the leaf areas at the time of application were measured, the average number of lesions per 1 $cm^2$ was determined, and the control titer was determined based thereon by the following equation.

Control titer = 100 - (average number of lesions of the treatment group)/(average number of lesions of the control group) × 100

The results are shown in Table 15. The control titer attained with the use of the culture solution of BAL bacteria was 89.7. This indicates that rice brown spot could be inhibited.

Table 15

| | Total area of leaves measured ($cm^2$) | Number of lesions | Number of lesions per 1$cm^2$ | Control titer |
|---|---|---|---|---|
| Treatment group (culture solution) | 264.1 | 102 | 0.39 | 89.7 |
| Control group (water) | 352.38 | 1316 | 3.73 | - |

Example 19

Effects of culture solution of BAL bacteria for inhibiting cucumber gray mold fungi *(Botrytis cinerea)*

The cucumber cotyledons were cut from the hypocotyl segments, and the cut cotyledons were placed in a container lined with a moistened paper towel so as to bring the cut surfaces into contact with the paper towel. *Botrytis cinerea* was allowed to grow on a potato decoction agar medium with 2% sucrose (PSA) in advance, and the spores generated under BLB application was suspended in a potato decoction agar medium with 2% sucrose (PG). The spore density in the suspension was adjusted to 2x $10^7$ spores/ml, 10 cotyledons were placed per region, 50 μl each of the suspension was applied dropwise to the center thereof, and a paper disk (Toyo Roshi, for testing antibiotics, thick, a diameter of 8 mm) was allowed to adhere thereto. Further, 50 μl of the culture solution of BAL bacteria or a control substance, i.e., distilled water or iprodione wettable powder (Rovral) (dilution factor: 1,000) was applied dropwise through the paper disk, the container was hermetically sealed and allowed to stand in the bright at 20°C for 16 hours.

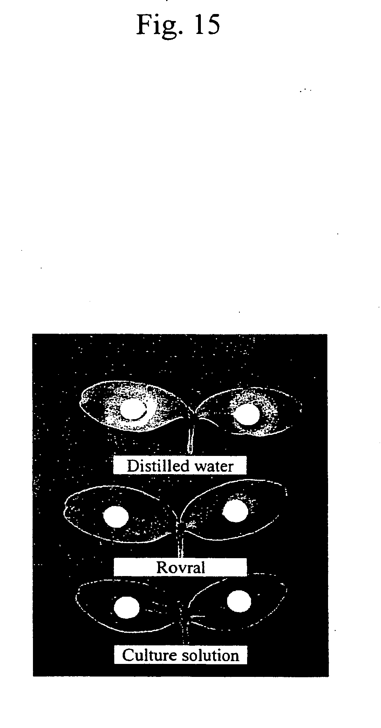

The results are shown in Table 16 and Fig. 15. The results are represented based on the browned diameter in the vicinity of the paper disk caused by infection. The control titer was determined in comparison with distilled water by the following equation.

Control titer = 100 - (average browned diameter of treatment group)/( average browned diameter of distilled water) × 100

Browning of the cotyledons 3 days after the application is shown in Fig. 15, and the average browned diameters among 10 cotyledons are shown in Table 16. Compared with the control group, the browned diameter of the plant to which the culture solution had been applied was apparently smaller. This indicates that cucumber gray mold was inhibited.

Table 16

| | Distilled water | Iprodione wettable powder | Culture solution |
|---|---|---|---|
| Average browned diameters (mm) | 19.6 | 11.2 | 3.3 |
| Control titer | - | 42.9 | 83.2 |

Example 20

Control of cucumber brown spot via ground application of culture solution of BAL bacteria

The cucumber plant (variety: Matsukaze), which had been forced to germinate on April 28, 2004, were grown in a greenhouse. A sufficient amount of the culture solution of BAL bacteria or a control substance, i.e., distilled water or Daconil 1000 (dilution factor: 1,000), was applied to the seedlings at the time of 2 true leaves development. After 5 hours had elapsed at 25°C and the applied solution was dried. Thereafter, a suspension of *Corynespora casiicola* spores (the stock strain of the Laboratory of Pathology and Biotechnology of the Musashino Seed Co., Ltd.) adjusted to a spore density of $10^5$ spores/ml was introduced by spraying. The suspension was prepared as follows: the spores were cultured in PSA medium in advance, distilled water was poured into a petri dish, and the flora was rubbed with a brush to wash the spores away, followed by filtration through two-ply gauze. After such application, the cucumber plant was covered with a plastic container to prepare a moist chamber at 25°C, the plant was allowed to stand therein for 24 hours, and it was supervised in the bright at 25°C for 16 hours. Twenty days after the application, the total number of lesions on the fully developed leaves at the time of application were measured, the average number of lesions per one leaf was determined, and the control titer was determined by the following equation.

$$\text{Control titer} = 100 - (\text{average number of lesions of treatment group})/(\text{average number of lesions of control group}) \times 100$$

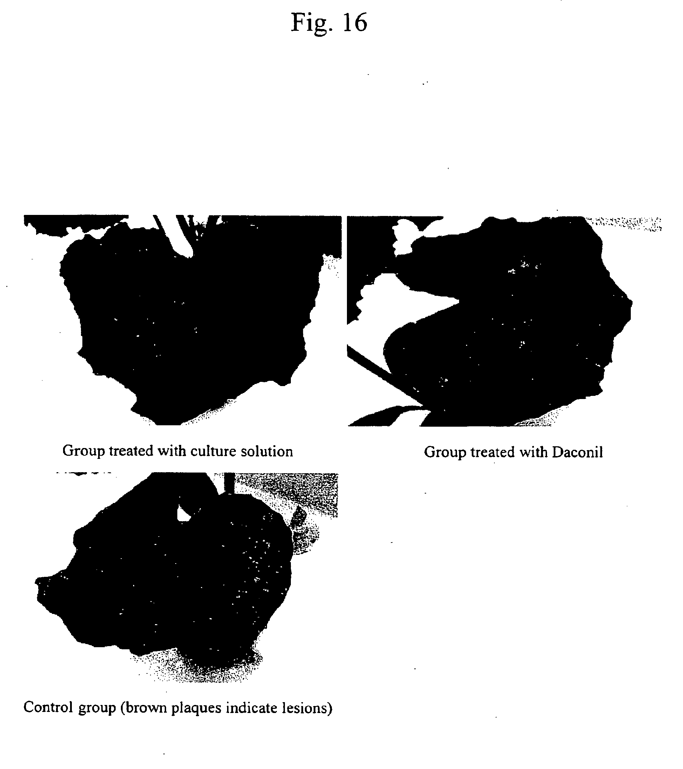

The results are shown in Table 17. In Table 17, the reference numerals 1-1, 1-2, 2-1, and 2-2 each independently represent the first leaf of the first plant, the second leaf of the first plant, the first leaf of the second plant, and the second leaf of the second plant, respectively. The appearances of the treatment groups 20 days after the initiation of the experiment are shown in Fig. 16. The control titer attained with the use of the culture solution of BAL bacteria and that attained with the use of Daconil 1000 were both 100. This indicates that cucumber brown spot could be inhibited by the use of the culture solution of BAL bacteria as with the use of Daconil 1000.

Table 17

| | Number of lesions | | | | | Control titer |
|---|---|---|---|---|---|---|
| | 1-1 | 1-2 | 2-1 | 2-2 | Average | |
| Culture solution of BAL bacteria | 0 | 0 | 0 | 0 | 0 | 100 |
| Daconil group | 0 | 0 | 0 | 0 | 0 | 100 |
| Control group (distilled water) | 53 | 68 | 74 | 5 | 50 | - |

Example 21

Test of effects of culture solution of BAL bacteria on black spot (*Alternaria brassicae*)

A platinum loopful of BAL bacteria was scraped off from YPA slant medium, introduced into 100 ml of bean curd refuse extraction medium filled in a 300-ml triangular flask, and subjected to shake culture using a reciprocal shaker having an amplitude of 10 cm at 120 rpm at 25°C for 4 days. This culture solution of BAL bacteria was uniformly sprayed onto the leaves of turnip (variety: Natsumaki No.13, Kokabu; date of application: March 10, 2004; degree of growth at the time of application: 3 foliage leaves, cultivated in a 75-mm black plastic pot for 20 days) using a small hand spray, and it was then allowed to stand in a moist chamber at 25°C for 24 hours. The spores of *Alternaria brassicae* that had been previously cultured on PSA plate (the stock strain of the Laboratory of Pathology and Biotechnology of the Musashino Seed Co., Ltd.) were suspended in distilled water. The resulting suspension was uniformly sprayed on the leaves of turnip using a small hand spray, it was placed in a moist chamber at 25°C for 24 hours, and it was supervised at 20°C until the symptoms were developed. At the time of application, the spore density in the suspension was adjusted by diluting the suspension so as to contain approximately 40 spores per field based on the 200x microscope. For comparison, a group in which water was applied to the leaves instead of the culture solution of BAL bacteria (the control group) and a group in which a 1000-fold solution of iprodione wettable powder as an existing control agent was sprayed to the leaves were provided. Investigation was carried out according to the criteria shown below, and 3 leaves of each plant were subjected to investigation.

0: Without symptoms
1: The area exhibiting symptoms accounts for 25% or smaller of the leaf area.
2: The area exhibiting symptoms accounts for 25% to 50% of the leaf area.
3: The area exhibiting symptoms accounts for 50% or larger of the leaf area.
4: Blighted

Based on the results of investigation, the incidence of disease was calculated by the following equation:

$$\text{incidence of disease} = (1n_1 + 2n_2 + 3n_3 + 4n_4)/(4 \times \text{total number of investigated plants})$$

wherein, $n_1$, $n_2$, $n_3$, and $n_4$ each independently represent the number of plants categorized by the above criteria.

Based on the calculated incidence of disease, the control titer was determined by the following equation.

$$\text{Control titer} = 100 - \text{incidence of disease of treatment group}/\text{incidence of disease of control group} \times 100$$

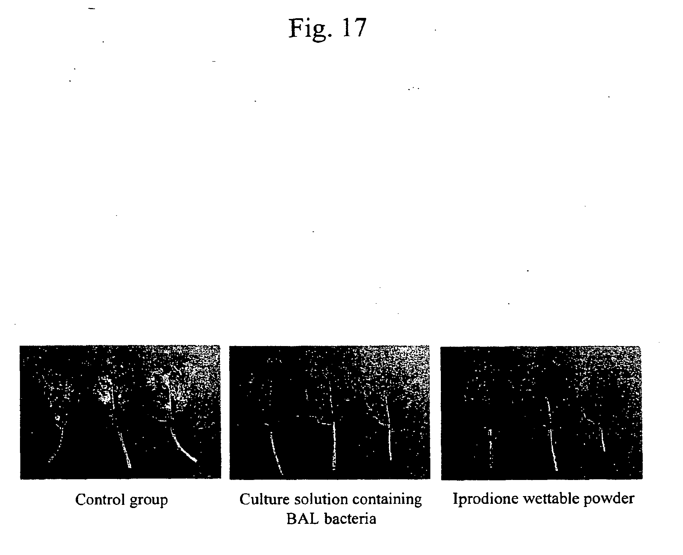

The results are shown in Table 18. Fig. 17 shows the appearance of the leaves at the time of incidence evaluation. As shown in the Table 18 and in Fig. 17, application of the culture solution of BAL bacteria resulted in the complete inhibition of black spot.

Table 18

| | Incidence of disease | Control titer |
|---|---|---|
| Water (control group) | 83.3 | - |
| Culture solution of BAL bacteria | 0.0 | 100.0 |
| 1000-fold iprodione solution | 0.0 | 100.0 |

Example 22

Tests of the effects of culture solution of BAL bacteria on white rust fungi (*Albugo macrospora*)

A platinum loopful of BAL bacteria was scraped off from YPA slant medium, introduced into 100 ml of bean curd refuse extract filled in a 300-ml triangular flask, and subjected to shake culture using a reciprocal shaker with an amplitude of 10 cm at 120 rpm at 25°C for 4 days. This culture solution of BAL bacteria was uniformly sprayed on the leaf surface of bok choy (variety: Fukushoumi; date of application: February 12, 2004; degree of growth at the time of application: 5 foliage leaves, cultivated in a 75-mm black plastic pot for 30 days) using a small hand spray, and the sprayed sample was then allowed to stand in a moist chamber at 25°C for 24 hours. The sample was allowed to stand at 25°C for an additional 24 hours, zoosporangia sampled from the conidial layer of the *Albugo macrospora* that had spontaneously occurred in *Brassica chinensis komatsuna* in a breeding nursery were then suspended in distilled water, the suspension was introduced to the sample by spraying with the use of a small hand spray, and the sprayed sample was allowed to stand in a moist chamber at 11°C for 24 hours. The zoosporangia concentration in the suspension at the time of application was diluted to contain approximately 30 zoosporangia per visual field of a 200x microscope. Until the symptom was developed, a tunnel and a heating house were used only after dark. For comparison, a group to which water was sprayed instead of the culture solution of BAL bacteria (the control group) was provided. The number of conidial layers per unit area of two leaf blades of each plant to which *Albugo macrospora* had been applied as described above was investigated. Based on the results of investigation, the control titer of the treatment group was calculated by the following equation.

$$\text{Control titer} = 100 - (\text{the number of conidial layers per 1 cm}^2 \text{ of the treatment group/ the number of conidial layers per 1 cm}^2 \text{ of the control group}) \times 100$$

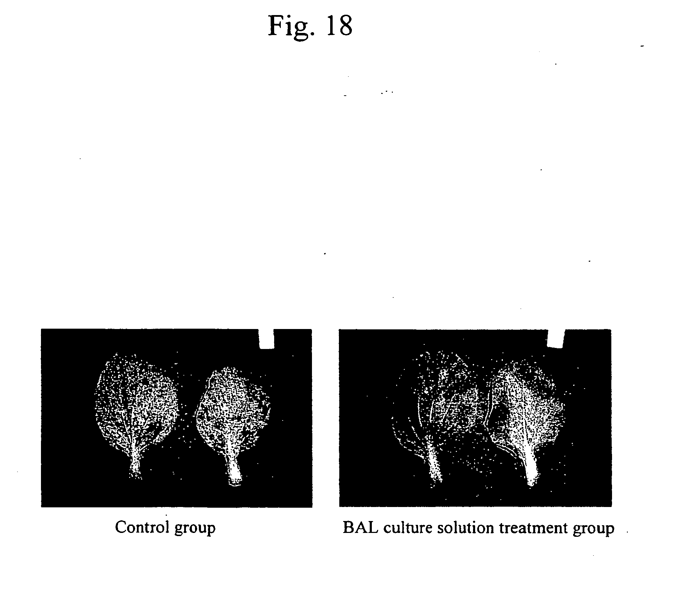

The results are shown in Table 19. Fig. 18 shows the appearance of the diseased leaf. The control titer attained by the application of the culture solution of BAL bacteria was high. This indicates that such application is effective and practical utility thereof is proven.

Table 19

| | Number of conidial layers/cm$^2$ | Control titer |
|---|---|---|
| Water (control group) | 112.0 | - |
| Culture solution of BAL bacteria | 0.515 | 99.5 |

Example 23

Test of effects of BAL bacteria on *Fusarium oxysporum f. sp. raphani*

The Ecobran material (40 g, commercialized by Chiba Seifun Co., Ltd., a bran component: 29% N, 38% $P_2O_5$, 2.0% $K_5O$, 52.5% organic matter, and 35% to 40% moisture; pH 7.5; the C/N ratio: 10) and 10 g of rice bran were thoroughly mixed, the mixture was subjected to high-pressure sterilization at 121°C for 1 hour, and high-pressure sterilization was carried out again under the same conditions 24 hours later. Sterilized water (30 ml) and the powdered culture product of BAL bacteria in bean curd refuse (0.6 g, see Example 6), which was diluted 10-fold with diatomaceous earth, were added to the sterilized material, they were thoroughly mixed and allowed to stand at 30°C for 4 days, and the resultant was designated as a material containing BAL bacteria. The *Fusarium oxysporum f. sp. raphani* (the stock strain of the Laboratory of Pathology and Biotechnology of the Musashino Seed Co., Ltd.) that had been precultured on PSA plate in advance was cut into 5-mm$^2$ pieces, introduced into 100 ml of PG medium filled in a 300-ml triangular flask, and then subjected to shake culture using a reciprocal shaker with an amplitude of 10 cm at 120 rpm at 25°C for 5 days. The culture solution was filtered through two-ply gauze and centrifuged at 3,000 rpm for 10 minutes to recover spores. These spores were suspended in distilled water and centrifuged under the same conditions to recover a sediment. This procedure was repeated two times for washing. The sediment was suspended in distilled water, and the resultant was homogenously incorporated into sterilized soil to prepare contaminated soil containing 2x10$^7$ spores/ml of *Fusarium oxysporum f. sp. raphani.* The obtained soil was allowed to stand at 25°C for 24 hours, the material containing BAL bacteria was thoroughly mixed therewith to a concentration of 500 kg/10a of the material (containing 1 x 10$^7$ cfu/g of BAL bacteria), the mixture was allowed to stand at 25°C for 6 days, turnip (variety: Natsumaki No. 13, kokabu) was introduced on May 20, 2004, and it was then supervised at 25°C. For comparison, sterilized soil, contaminated soil containing no material containing BAL bacteria (a control group), and a chemical control agent, which was to be introduced into the contaminated soil, followed by perfusion of 3 l/m$^2$ of 1000-fold solution of benomyl wettable powder, were also tested. The degree of yellow blight of the cotyledon 18 days after the application was investigated. Based on the results of investigation, the incidence of disease was calculated by the following equation.

$$\text{Incidence of disease} = \text{number of diseased strains / total number of investigated stocks.}$$

Based on the calculated incidence of disease, the control titer was determined by the following equation.

$$\text{Control titer} = 100 - \text{incidence of disease of the treatment group/incidence of disease of the control group} \times 100$$

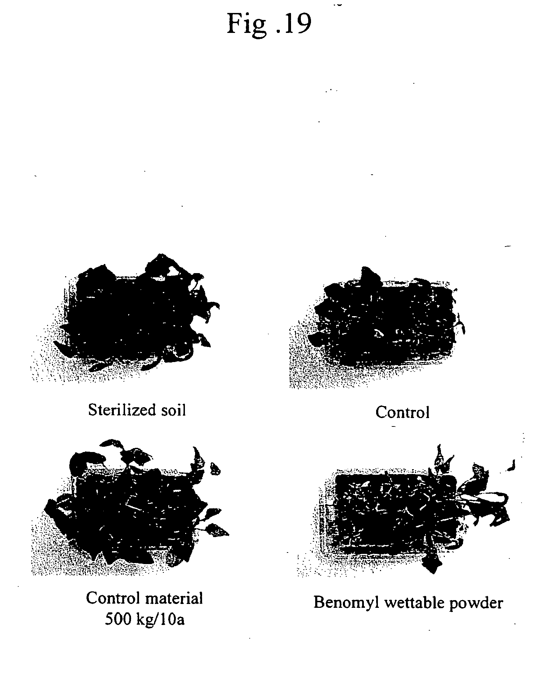

The results are shown in Table 20. Fig. 19 shows the appearance of samples 18 days after the initiation of the experiment. The group to which the control material containing the culture solution of BAL bacteria had been applied exhibited a higher potency of inhibiting the disease than the group to which a chemical control agent had been applied.

Table 20

| | Number of diseased strains | | Incidence of disease | Control titer |
|---|---|---|---|---|
| | - | + | | |
| Sterilized soil | 30 | 0 | 0.0 | - |
| Contaminated soil (control group) | 4 | 26 | 86.7 | - |
| Benomyl wettable powder | 6 | 23 | 79.3 | 8.5 |
| Control material (500-kg/10a) | 15 | 13 | 46.4 | 46.5 |
| * "-" in number of diseased strains: symptoms appeared; "+" therein: no symptoms | | | | |

Example 24

Test of effects of BAL bacteria on seedling damping-off fungus (*Rhizoctonia solani*)

The Ecobran material (40 g, described in Example 13) and 10 g of rice bran were thoroughly mixed, subjected to high-pressure sterilization at 121°C for 1 hour, and subjected to high-pressure sterilization again under the same conditions 24 hours later. Sterilized water (30 ml) and the powdered culture product of BAL bacteria in bean curd refuse (0.6 g, see Example 6), which were diluted 10-fold with diatomaceous earth, were added to the sterilized material, they were thoroughly mixed and allowed to stand at 30°C for 7 days, and the resultant was designated as a material containing BAL bacteria. This material was incorporated into sterilized soil to reach the amount thereof to 1 t/10a ($5 \times 10^7$ cfu/g of BAL bacteria in the soil) and allowed to stand in the dark at 25°C for 10 days. The seedling dampling-off fungi that had been cultured on PSA plate for 2 days (*Rhizoctonia solani* AG-4, IIIA, seedling dampling-off fungus, the stock strain of the Laboratory of Pathology and Biotechnology of the Musashino Seed Co., Ltd.) were punched out using a cork borer, and 3 pieces of the floras were placed on the bottom surface of the ice cream cup (length: 7 cm; width: 7 cm; and height 5 cm) at the same intervals. Sterilized soil (125 ml) containing the material was filled so as to cover the flora pieces, 100 mg of bentgrass (Yukijirusi) was introduced, and it was supervised in an artificial climate chamber at 28°C. For comparison, a group in which a sterilized soil into which neither BAL bacteria nor seedling dampling-off fungi was introduced was employed (the sterilized soil group), and a group in which a sterilized soil into which BAL bacteria was not introduced and seedling dampling-off fungi was introduced was employed (the control group) were provided. Regarding these groups, introduction and supervision were carried out in the same manner as stated above unless otherwise noted. The number of survived strains was inspected 11 days after the BAL bacteria application, the number of survived strains in the sterilized soil was designated as a common number of strains to which BAL bacteria had been applied in each test group, and the ratio of survived strains and the ratio of blighted strains were determined by the following equations.

$$\text{Ratio of survived strains} = \text{number of survived strains / number of survived strains in sterilized soil} \times 100$$

$$\text{Ratio of blighted strains} = 100 - \text{ratio of survived strains}$$

Based on the ratio of blighted strains, the control titer was further determined by the following equation.

$$\text{Control titer} = 100 - (\text{ratio of blighted strains of the treatment group} / \text{ratio of blighted strains of the control group}) \times 100$$

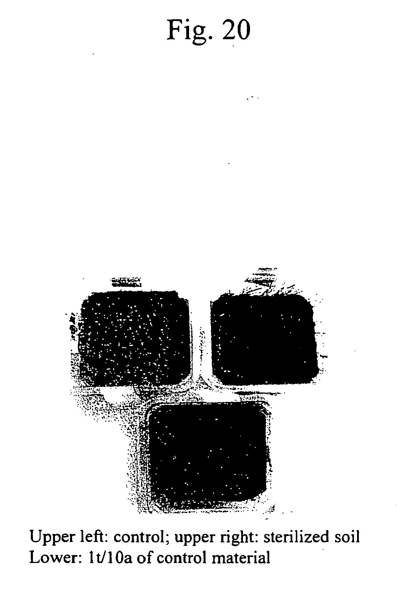

The results are shown in Table 21. Fig. 20 shows the appearance of samples 11 days after the initiation of the experiment. Although the control titer was low due to an extremely high degree of contamination, the effects were observed.

Table 21

| | Ratio of survived strains (%) | Ratio of blighted strains (%) | Control titer |
|---|---|---|---|
| Sterilized soil | 100 | 0 | - |
| Control | 3 | 97 | - |
| Control material (1 t/10a) | 30 | 70 | 27.8 |

Example 25

Inhibition of *Tobacco mosaic virus* (TMV) infection in TMV-infected soil with the use of a control material containing BAL bacteria

TMV-infected leaves (4 g, wet weight) were introduced into a mortar and ground in 10 ml of 1/15M phosphate buffer (pH 6.98) to prepare a sap for application. This sap was aliquoted to two 50-ml Falcon tubes and a phosphate buffer was added to bring the amount of the solution to 50 ml. Dehydrated disinfected soil (100 ml), 1.25 g of rice bran (equivalent of 500 kg/10a), and 50 ml of the sap were introduced to a 500-ml beaker. As a BAL bacteria treatment group, BAL bacteria were isolated by centrifuging 20 ml of the culture solution that had been shake-cultured in a bean curd refuse extract at 3000 rpm for 30 minutes, the isolated BAL bacteria were suspended in 50 ml of phosphate buffer, and the resulting suspension was introduced into the above soil, followed by thorough mixing with the use of a glass rod. As a control group, 50 ml of phosphate buffer was mixed. The soil surface was covered with vinyl, the beaker was wrapped, the opening was strapped to carry out a simple solar technique, and the beaker was allowed to stand in an incubator at 38°C. Four days after the initiation of the process, 3 g each of soil was sampled from each treatment group and introduced into the beaker, the beaker was allowed to stand, and the supernatant was introduced into *Nicotiana glutinosa,* which is a TMV differential plant species and which had established 8 true leaves, with the aid of carborundum powders (*Shokubutu byourigaku jikkenhou* (*plant pathological experimen*), Shoji Sato, Masao Goto, and Yoji Doi (ed.), Kodansha Ltd.). *Nicotiana glutinosa* was supervised in an artificial climate chamber at 20°C, the number of local lesions, which appeared on the leaves 6 days after the application, was counted, the leaf area was measured, and the control titer was determined by the following equation based on the number of local lesions per unit area.

$$\text{Control titer} = 100 - (\text{number of lesions per unit area of the treatment group} / \text{number of lesions per unit area of the control group}) \times 100$$

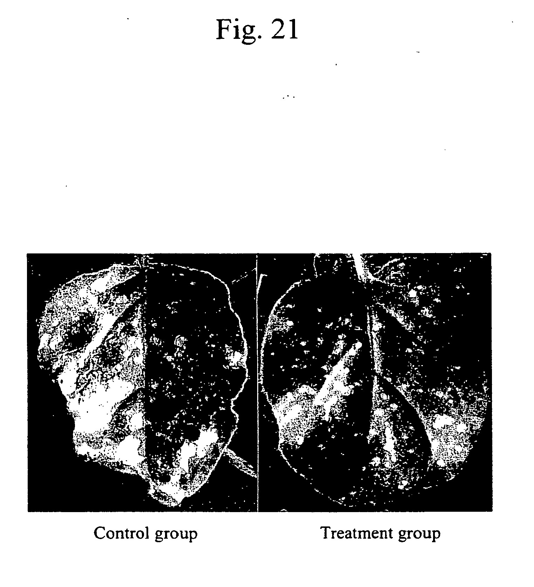

The results are shown in Table 22. Fig. 21 shows the appearance of samples upon disease development.

Table 22

| | Inoculated leaves | Number of local lesions | Leaf area | Number of lesions per 1 $cm^2$ | Average | Control titer |
|---|---|---|---|---|---|---|
| Control | 1 | 246 | 26.67 | 9.2 | 9.6 | - |
| | 2 | 283 | 21.29 | 13.3 | | |
| | 3 | 174 | 27.51 | 6.3 | | |

(continued)

| | Inoculated leaves | Number of local lesions | Leaf area | Number of lesions per 1 $cm^2$ | Average | Control titer |
|---|---|---|---|---|---|---|
| Treated with BAL bacteria | 1 | 166 | 28.6 | 5.8 | 4.4 | 54.0 |
| | 2 | 135 | 25.64 | 5.3 | | |
| | 3 | 70 | 32.03 | 2.2 | | |

Example 26

Inhibition of *Melon necrotic spot virus* infection

Twenty melon seeds (variety: Earl's Miyabi) were introduced to a 9-cm petri dish, which was lined with a filter paper and filled with 4.25 ml of distilled water, and they were forced to germinate at 25°C for 2 days. A 25-well cell was filled with 50 ml of Kureha garden soil, the the germinated seeds were put on the soil, and then the cell was covered with 50 ml of sterilized soil. Seedlings were raised in an artificial climate chamber at 25°C. The inoculum used in the test was prepared by introducing 2 g of the melon necrotic spot virus-infected leaves (the origin of the virus: the field in Isahaya, Nagasaki, Japan) that had been cryopreserved at -30°C into a mortar, and grinding the leaves in 10 ml of 1/15M phosphate buffer (pH 6.98). The resulting ground homogenate was filtered through two-ply gauze and then subjected to the following test.

The ground homogenate (3 ml) was mixed with 3 ml of the culture solution of BAL bacteria that had been shake-cultured using bean curd refuse medium for 8 days, the container was sealed with Parafilm, and it was then allowed to stand in an incubator at 25°C. This was introduded into melon cotyledons with the aid of carborundum powder 5 and 22 hours later (in accordance with Example 25). As a control group, a group to which the ground homogenate and a phosphate buffer had been applied was provided. After application, the plants were supervised in an artificial climate chamber at 25°C. The number of local lesions appeared on the cotyledons 7 or 8 days after the application was counted, the number of lesions per cotyledon was calculated, and the control titer was determined by the following equation. The control titers 5 hours after the mixture was 73.3, and that 22 hours after the mixture was 80.

Control titer = 100 - (number of lesions per cotyledon of the treatment group/ number of lesions per cotyledon of the control group) × 100

The results are shown in Table 23. Fig. 21 shows the appearance of samples upon disease development.

Table 23

| | Mixing duration | Number of local lesions appeared on cotyledon | | | | | | | | | | Number of lesions per cotyledon | Control titer |
|---|---|---|---|---|---|---|---|---|---|---|---|---|---|
| | | Strain 1 | | Strain 2 | | Strain 3 | | Strain 4 | | Strain 5 | | | |
| Phosphate buffer (control group) | 5 hours | 4 | 2 | 8 | 2 | 9 | 4 | 10 | 18 | | | 7.1 | - |
| | 22 hours | 8 | 12 | 12 | 7 | 4 | 7 | 10 | 6 | 7 | 7 | 8.0 | - |
| Culture solution of BAL bacteria | 5 hours | 2 | 3 | 2 | 3 | 1 | 0 | 1 | 1 | 2 | 4 | 1.9 | 73.3 |
| | 22 hours | 2 | 2 | 5 | 1 | 1 | 3 | 0 | 0 | 2 | 0 | 1.6 | 80.0 |

Industrial Applicability

According to the present invention, plant diseases resulting from infection with or proliferation of plant pathogenic bacteria, fungi, or viruses can be biologically controlled.

## Claims

1. A bacterial strain of the genus *Bacillus,* DAIJU-SIID2550 (accession number: FERM BP-10114).

2. A control agent or material for plant diseases comprising bacteria of the genus *Bacillus* capable of inhibiting infection with or proliferation of plant pathogenic bacteria, fungi, or viruses, wherein the bacteria of the genus *Bacillus* are of the DAIJU-SIID2550 strain (accession number: FERM BP-10114).

3. A method for controlling plant diseases comprising application of the control agent or material according claim 2 to a host plant of the plant pathogenic bacteria, fungi, or viruses.

4. The method according to claim 3, wherein the plant pathogenic bacteria are of the genus *Agrobacterium, Clavibacter, Erwinia, Pseudomonas, Ralstonia, Corynebacterium, Curtobacterium, Burkholderia,* or *Xanthomonas,* and the plant diseases are caused thereby.

5. The method according to claim 3, wherein the plant pathogenic fungi are airborne or soil-borne fungi, and the plant diseases are caused thereby.

6. The method according to claim 3, wherein the plant pathogenic viruses are *Tobacco mosaic virus, Pepper mild*

*mottle virus, Tomato mosaic virus, Melon necrotic spot virus, Cucumber green mottle mosaic virus,* or *Kyuri green mottle mosaic virus,* and the plant diseases are caused thereby.

**7.** The method according to claim 3, wherein the plant belongs to *Brassicaceae, Solanaceae, Cucurbitaceae, Liliaceae, Leguminosae, Asteraceae, Chenopodiaceae, Gramineae, Rosaceae, Caryophyllaceae, Primulaceae, Rutaceae, Vitaceae, Actinidiaceae, Ebenaceae, Umbelliferae, Convolvulaceae,* or *Araceae.*

**8.** The method according to claim 3, wherein the control agent or material is applied to plants by at least one process selected from the group of processes consisting of coating plant seeds with the bacteria, soaking plant seeds in a suspension containing the bacteria, perfusion of the bacteria in soil for plant cultivation, incorporation of the bacteria in soil for plant cultivation, spraying the bacteria on plant stem or foliage, and contact of the bacteria with plant damages.

## Patentansprüche

**1.** Bakterienstamm der Gattung Bacillus, DAIJU-SIID2550 (Zugangsnr.: FERM BP-10114).

**2.** Bekämpfungsmittel oder -material für Pflanzenerkrankungen, das Bakterien der Gattung Bacillus enthalten, die in der Lage sind, Infektion mit oder die Verbreitung von pflanzenpathogenen Bakterien, Pilzen oder Viren zu verhindern, worin die Bakterien der Gattung Bazillus vom DAIJU-SIID2550-Stamm (Zugangsnr.: FERM BP-10114) stammen.

**3.** Verfahren zur Bekämpfung von Pflanzenerkrankungen, welches die Anwendung des Bekämpfungsmittels oder -materials nach Anspruch 2 an einer Wirtspflanze der pflanzenpathogenen Bakterien, Pilze oder Viren umfassen.

**4.** Verfahren nach Anspruch 3, worin die pflanzenpathogenen Bakterien von der Gattung Agrobacterium, Clavibacter, Erwinia, Pseudomonas, Ralstonia, Corynebacterium, Curtobacterium, Burkholderia oder Xanthomonas stammen und die Pflanzenerkrankungen **dadurch** verursacht werden.

**5.** Verfahren nach Anspruch 3, worin die pflanzenpathogenen Pilze in der Luft befindliche oder im Boden befindliche Pilze sind und die Pflanzenerkrankungen **dadurch** verursacht werden.

**6.** Verfahren nach Anspruch 3, worin die pflanzenpathogenen Viren Tabakmosaikvirus, Mildes Paprikascheckungsvirus, Tomatenmosaikvirus, Nekrotisches Melonenfleckenvirus, Gurkengrün-Scheckungsmosaikvirus oder Kyurigrün-Scheckungsmosaikvirus sind und die Pflanzenerkrankungen **dadurch** verursacht werden.

**7.** Verfahren nach Anspruch 3, worin die Pflanze zu Brassicaceae, Solanaceae, Cucurbitaceae, Liliaceae, Leguminosae, Asteraceae, Chenopodiaceae, Gramineae, Rosaceae, Caryophyllaceae, Primulaceae, Rutaceae, Vitaceae, Actinidiaceae, Ebenaceae, Umbelliferae, Convolvulaceae oder Araceae gehört.

**8.** Verfahren nach Anspruch 3, worin das Bekämpfungsmittel oder -material auf Pflanzen mit Hilfe von zumindest einem Verfahren angewendet wird, das aus der Gruppe von Verfahren ausgewählt ist, die aus der Beschichtung von Pflanzensamen mit den Bakterien, dem Einweichen von Pflanzensamen in einer Suspension, welche die Bakterien enthält, der Perfusion der Bakterien in den Boden zur Pflanzenzüchtung, dem Einbetten der Bakterien in den Boden zur Pflanzenzüchtung, dem Besprühen der Pflanzenstängel oder -blätter mit den Bakterien und dem Kontaktieren der Bakterien mit den Pflanzenschäden besteht.

## Revendications

**1.** Souche bactérienne du gène *Bacillus,* DAIJU-SIID2550 (numéro d'accès: FERM BP-10114).

**2.** Agent ou matériau de contrôle de maladies de plantes comprenant une bactérie du gène *Bacillus* apte à inhiber l'infection avec ou la prolifération de bactéries, champignons ou virus pathogènes des plantes, où les bactéries du gène *Bacillus* sont de la souche DAIJU-SIID2550 (numéro d'accès: FERM BP-10114).

**3.** Méthode de contrôle de maladies de plantes comprenant l'application d'un agent ou matériau de contrôle selon la revendication 2 à une plante hôte de bactéries, champignon ou virus pathogènes des plantes.

**4.** Méthode selon la revendication 3, dans laquelle les bactéries pathogènes de plantes sont du gène *Agrobacterium, Clavibacter, Erwinia, Pseudomonas, Ralstonia, Corynebacterium, Curtobacterium, Burkholderia* ou *Xanthomonas,* et les maladies des plantes sont provoquées par celles-ci.

**5.** Méthode selon la revendication 3, dans laquelle les champignons pathogènes des plantes sont des champignons transportés par l'air ou telluriques, et les maladies des plantes sont provoquées par ceux-ci.

**6.** Méthode selon la revendication 3, dans laquelle les virus pathogènes des plantes sont *Tobacco mosaic virus, Pepper mild mottle virus, Tomato mosaic virus, Melon necrotic spot virus, Cucumber green mottle mosaic virus ou Kyuri green mottle mosaic virus,* et les maladies des plantes sont provoquées par ceux-ci.

**7.** Méthode selon la revendication 3, dans laquelle la plante appartient à *Brassicaceae, Solanaceae, Cucurbitaceae, Liliaceae, Leguminosae, Asteraceae, Chenopodiaceae, Gramineae, Rosaceae, Caryophyllaceae, Primulaceae, Rutaceae, Vitaceae, Actinidiaceae, Ebenaceae, Umbelliferae, Convolvulaceae ou Araceae.*

**8.** Méthode selon la revendication 3, dans laquelle l'agent ou le matériau de contrôle est appliqué aux plantes par au moins un processus sélectionné dans le groupe de processus consistant à recouvrir les semences des plantes de bactéries, à tremper les semences des plantes dans une suspension contenant les bactéries, une perfusion des bactéries dans le sol pour la culture des plantes, l'incorporation des bactéries dans le sol pour la culture des plantes, la pulvérisation des bactéries sur des tiges ou feuilles des plantes et le contact des bactéries avec des endommagements des plantes.

Fig. 1

Fig. 2

Fig. 3

Fig. 4

Fig. 5

Fig. 6

Fig. 7A

Lannate
Admire
Mospilan
Padan
Elsan
Actara
Torebon
Kotetsu
Jimandaisen
Amistar
Daconil
Sthana
Benlate
Z-bordeaux
Ridomyl MZ
Bestguard

Fig. 7B

Affirm
DDVP

Rizolex
Rovral

Fig. 8

AG medium containing *Bacillus* bacteria

AG medium

Fig. 9

AG medium containing *Bacillus* bacteria
AG medium
$10^5$
$10^6$
$10^7$

Fig. 10

AG medium containing *Bacillus* bacteria
AG medium
$10^6$
$10^7$

Fig. 11

Section with soft rot (+) Section without soft rot (-)

Fig. 12A

Soaking for 48 hours Without soaking

Fig. 12B

Soaking for 48 hours Without soaking

Fig. 13

Stock solution (2-fold)

Distilled water

5-fold diluent (10-fold)

Fig. 14

Group treated with stock solution

Group treated with 5-fold diluent

Group treated with 10-fold diluent

Group treated with distilled water (control group)

Fig. 15

Distilled water
Rovral
Culture solution

Fig. 16

Group treated with culture solution

Group treated with Daconil

Control group (brown plaques indicate lesions)

Fig. 17

Control group

Culture solution containing BAL bacteria

Iprodione wettable powder

Fig. 18

Control group

BAL culture solution treatment group

Fig .19

Sterilized soil

Control

Control material
500 kg/10a

Benomyl wettable powder

Fig. 20

Upper left: control; upper right: sterilized soil
Lower: 1t/10a of control material

Fig. 21

Control group Treatment group

Fig. 22

Local lesions

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5344647 A
- US 5061495 A
- JP 8175919 A
- JP 2003277210 A
- JP 2002145712 A
- JP 9124427 A
- JP 11302120 A
- US 5935571 A
- JP 7267811 A
- JP 2004055059 A
- JP 2004216083 A


### Non-patent literature cited in the description


- **Phae, C. G. ; Shoda, M. ; Kita, N. ; Nakano, M. ; Ushiyama, K.** *Ann. Phytopath. Soc. Japan,* 1992, vol. 58, 329-339
- **Masao Yamada.** Biseibutsu nouyaku - kankyou hozenkata nougyou wo mezasite. *Zenkoku nouson kyouiku kyoukai,* 2000, 328
- Shokubutsu boueki kouza-byougai-hen. Japan Plant Protection Association, 1983, 281
- **Randhawa, P. S. ; Schaad, N. W.** *Phytopathology,* 1984, vol. 74, 268-272

SEQUENCE LISTING

<110> ITSUKI Co., Ltd.

<120> Method and agent for treating and preventing plant deseases by using bacterium belonging to the genus of Bacillus

<130> PH-2257-PCT

<150> JP 2004-55059
<151> 2004-02-27

<150> JP 2004-216083
<151> 2004-07-23

<160> 1

<170> PatentIn version 3.1

<210> 1
<211> 1545
<212> DNA
<213> Bacillus sp.

<400> 1

tggagagttt gatcctggct caggacgaac gctggcggcg tgcctaatac atgcaagtcg 60

agcggacaga tgggagcttg ctccctgatg ttagcggcgg acgggtgagt aacacgtggg 120

```
taacctgcct gtaagactgg gataactccg ggaaaccggg gctaataccg gatggttgtc    180

tgaacygcat ggttcagaca taaaaggtgg cttyggctac cacttacaga tggacccgcg    240

gcgcattagc tagttggtga ggtaacggct caccaaggcg acgatgcgta gccgacctga    300

gagggtgatc ggccacactg ggactgagac acggcccaga ctcctacggg aggcagcagt    360

agggaatctt ccgcaatgga cgaaagtctg acggagcaac gccgcgtgag tgatgaaggt    420

tttcggatcg taaagctctg ttgttaggga agaacaagtg ccgttcaaat agggcggcac    480

cttgacggta cctaaccaga aagccacggc taactacgtg ccagcagccg cggtaatacg    540

taggtggcaa gcgttgtccg gaattattgg gcgtaaaggg ctcgcaggcg gtttcttaag    600

tctgatgtga aagcccccgg ctcaaccggg gagggtcatt ggaaactggg gaacttgagt    660

gcagaagagg agagtggaat tccacgtgta gcggtgaaat gcgtagagat gtggaggaac    720

accagtggcg aaggcgactc tctggtctgt aactgacgct gaggagcgaa agcgtgggga    780

gcgaacagga ttagataccc tggtagtcca cgccgtaaac gatgagtgct aagtgttagg    840

gggtttccgc cccttagtgc tgcagctaac gcattaagca ctccgcctgg ggagtacggt    900

cgcaagactg aaactcaaag gaattgacgg gggcccgcac aagcggtgga gcatgtggtt    960

taattcgaag caacgcgaag aaccttacca ggtcttgaca tcctctgaca atcctagaga   1020
```

```
taggacgtcc ccttcggggg cagagtgaca ggtggtgcat ggttgtcgtc agctcgtgtc    1080
gtgagatgtt gggttaagtc ccgcaacgag cgcaaccctt gatcttagtt gccagcattc    1140
agttgggcac tctaaggtga ctgccggtga caaaccggag gaaggtgggg atgacgtcaa    1200
atcatcatgc cccttatgac ctgggctaca cacgtgctac aatggacaga acaaagggca    1260
gcgaaaccgc gaggttaagc caatcccaca aatctgttct cagttcggat cgcagtctgc    1320
aactcgactg cgtgaagctg gaatcgctag taatcgcgga tcagcatgcc gcggtgaata    1380
cgttcccggg ccttgtacac accgcccgtc acaccacgag agtttgtaac acccgaagtc    1440
ggtgaggtaa cctttatgga gccagccgcc gaaggtggga cagatgattg gggtgaagtc    1500
gtaacaaggt agccgtatcg gaaggtgcgg ctggatcacc tcctt                     1545
```